(19) **Europäisches Patentamt / European Patent Office / Office européen des brevets**

(11) **EP 3 697 478 B1**

(12) **FASCICULE DE BREVET EUROPEEN**

(45) Date de publication et mention
de la délivrance du brevet:
**02.07.2025 Bulletin 2025/27**

(21) Numéro de dépôt: **18786345.1**

(22) Date de dépôt: **16.10.2018**

(51) Classification Internationale des Brevets (IPC):
*A45D 34/00* (2006.01)     *A61M 5/315* (2006.01)
*A45D 40/04* (2006.01)     *A61M 5/24* (2006.01)
*A61M 5/34* (2006.01)     *A61M 5/46* (2006.01)
*A61M 5/32* (2006.01)     *A61J 1/06* (2006.01)

(52) Classification Coopérative des Brevets (CPC):
**A61M 5/31501; A45D 34/00; A45D 40/04;
A61J 1/062; A61M 5/24; A61M 5/2448;
A61M 5/31525; A61M 5/3155; A61M 5/31576;
A61M 5/31583; A61M 5/31586; A61M 5/329;
A61M 5/3293; A61M 5/34; A61M 5/343;** (Cont.)

(86) Numéro de dépôt international:
**PCT/EP2018/078287**

(87) Numéro de publication internationale:
**WO 2019/076921 (25.04.2019 Gazette 2019/17)**

(54) **DISPOSITIF D'INJECTION MANUELLE**

MANUELLE INJEKTIONSVORRICHTUNG

MANUAL INJECTION DEVICE

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **17.10.2017 FR 1759714**

(43) Date de publication de la demande:
**26.08.2020 Bulletin 2020/35**

(60) Demande divisionnaire:
**23187807.5 / 4 252 802**

(73) Titulaire: **Edix SA**
**1331 Luxemburg (LU)**

(72) Inventeurs:
• **CHERIF-CHEIKH, Roland**
**08860 Castelldefels-Barcelona (ES)**

• **BOURGOIS, Tabatha**
**08002 Barcelona (ES)**
• **PARETA BELTRAN, Lluis**
**08755 Castellbisbal-Barcelona (ES)**
• **LACOMBE, Frederic**
**08173 Sant Cugat del Valles (ES)**
• **LACHAMP, Laurence**
**08850 Gava Barcelona (ES)**

(74) Mandataire: **Bourgouin, André et al**
**Grosset-Fournier & Demachy**
**54, rue Saint Lazare**
**75009 Paris (FR)**

(56) Documents cités:
EP-A1- 0 709 040     WO-A1-2007/017052
WO-A1-2009/153132     DE-A1- 102013 212 325
FR-A1- 2 953 415     US-A- 3 599 681
US-A- 4 493 348     US-A1- 2005 274 746
US-A1- 2011 052 303     US-B2- 8 062 254

**(Cont. page suivante)**

(52)  Classification Coopérative des Brevets (CPC):
(Cont.)A45D 2200/055; A61M 5/2466;
A61M 5/3202; A61M 5/46; A61M 2005/2411;
A61M 2005/2488

## Description

La présente invention concerne des perfectionnements aux dispositifs d'injection de type manuel en vue d'améliorer notamment à la fois leur facilité et leur précision d'utilisation.

On entendra ci-après par dispositifs d'injection manuels les dispositifs d'injection, par exemple les seringues ou les outils équivalents, destinés à l'administration des traitements médicaux tels que ceux délivrés en intradermique, en sous-cutané, en intramusculaire, en intraveineux, mais aussi en topique, transcutané ou par toute autre voie d'administration connue.

On sait que de nombreux traitements nécessitent l'administration de produits par injection. Il s'agit essentiellement de traitements thérapeutiques ou prophylactiques, mais aussi d'autres applications comme certains traitements esthétiques, cosmétiques, dermatologiques, dentaires ou orthopédiques. En dehors de l'utilisation chez l'humain, les injections trouvent aussi de plus en plus d'applications dans le domaine des traitements vétérinaires.

De manière générale, le secteur médical est confronté à une tendance allant vers l'expansion des besoins en matière de traitements injectables. De même, il est à prévoir, dans un futur proche, une recrudescence de la demande de solutions novatrices pour pratiquer ces injections, afin d'améliorer le confort de l'utilisateur et répondre aux problèmes nouveaux posés par l'injection de produits toujours plus complexes et moins faciles à administrer en raison notamment de leurs viscosités élevées, de leurs hautes concentrations, ou des problèmes de stabilité avant ou lors de leur injection.

Par ailleurs, on sait que de plus en plus de traitements sont chroniques et beaucoup sont, ou pourraient l'être, avantageusement administrés hors de l'hôpital voire chez le patient. Il est ainsi important d'améliorer l'expérience vécue par ce dernier lors des injections qu'il est amené à se faire pour assurer l'observance de son traitement, d'autant qu'il est connu du corps médical qu'une proportion importante de patients ne suit pas totalement ses prescriptions pour des raisons allant de la peur des piqures d'aiguilles, la douleur à l'injection ou alors jusqu'à une carence de site d'administration sain pour injecter quotidiennement leurs traitements.

Diverses études ont été menées pour analyser les attentes des patients susceptibles de définir les possibles améliorations dans le domaine des injections, ainsi que les aspects sur lesquels ces améliorations peuvent porter.

Ces études ont montré que les besoins essentiels peuvent être synthétisés ainsi qu'exposé ci-après :

- Injecter manuellement avec une plus grande force et un meilleur contrôle de cette force et de la vitesse d'injection, même pour des produits très visqueux ou pâteux, et pouvoir ainsi utiliser des aiguilles plus fines que celles que nécessitent les injections manuelles habituelles du type utilisé sur les seringues ou autres dispositifs d'injection,

- Améliorer la précision et le contrôle des dispositifs d'injection manuels surtout pour les volumes et vitesses d'injection plus faibles ou plus difficilement contrôlables avec les dispositifs d'injection manuels actuels, comme par exemple de type seringue,

- Mieux définir les sites d'injection et la stabilité de l'aiguille insérée même avec des temps d'implantation plus long et/ou avec des mécanismes d'injection différents de ceux des injections manuelles de type seringue,

- Pouvoir réaliser cela avec les contenants classiques et les procédés de fabrication habituels utilisés pour les dispositifs d'injection manuelle, par exemple de type seringue.

Afin de satisfaire ces besoins essentiels, on a proposé de multiples dispositifs qui cherchent à améliorer la prise en main des dispositifs d'injection, à réduire la taille des aiguilles, à maitriser la vitesse d'administration ou encore à contrôler les forces d'injection, certains constituant des solutions alternatives aux seringues tels que par exemple les pompes d'injection, les auto-injecteurs, les stylo-injecteurs etc...

On sait également que pour pratiquer ou contrôler une injection à partir d'un réservoir fermé par un piston mobile, les moyens faisant appel à des mécanismes d'injection par rotation sont largement éprouvés ainsi que décrit notamment dans le brevet US 2 283 915.

On a par ailleurs proposé dans le brevet US 4 189 065 un mécanisme d'injection par rotation faisant appel à une tige de piston filetée dont l'entraînement en rotation dans un écrou solidaire d'une seringue provoque le déplacement longitudinal du piston dans la seringue.

La demande de brevet français FR 2 953 415 propose un dispositif d'éjection pour éjecter des faibles doses d'un produit liquide ou pâteux

La demande internationale WO 2007/017052 concerne un mécanisme permettant d'éviter de régler une dose dépassant la quantité de médicament dans un réservoir d'un dispositif d'injection.

La demande de brevet américain US 2011/052303 décrit un appareil d'hygiène personnelle qui comprend une interface et un applicateur pour réguler l'alimentation de l'applicateur en solution d'assainissement.

La demande brevet européen EP 0 709 040 décrit un distributeur comprenant un récipient pour stocker un produit chimique distribuable tel qu'une composition pour les aisselles.

La demande de brevet américain US 2005/274746 décrit un emballage de distribution pour un produit cosmétique, antitranspirant, déodorant ou tout autre produit en bâton.

La demande de brevet allemand DE 10 2013 212325 décrit un dispositif pour régler le débit d'un système de distribution de fluide médical

Le brevet américain US 8 062 254 décrit une seringue orale ajustable comportant un ressort pour l'administration de médicaments.

On notera que pour des spécialistes médecins et infirmiers, une injection se fait par translation, alors que pour l'ensemble de la population, ce geste est beaucoup moins courant, naturel et simple que la rotation. En effet, l'injection en translation est un geste atypique qui n'est jamais réalisé dans la vie de tous les jours. Ce geste nécessite un positionnement précis de trois doigts. Cette translation peut donner au patient l'impression d'introduire quelque chose dans son corps par le déplacement du pouce en pression vers la peau. Ce geste réalisé sans appui fixe pose un problème de stabilité qui entraîne des mouvements de l'aiguille implantée et contrarie la précision des dosages. Ce geste est sensible à la pression reçue en retour, qui peut être perçue comme variable, ce qui le rend donc difficile à réaliser à vitesse constante du début à la fin. Finalement, dans cette position de maintient instable, des pauses lors de l'injection en fonction de la douleur ressentie sont difficiles à réaliser.

L'injection en rotation reprend un geste habituel réalisé quotidiennement dans la vie de tous les jours, qui peut être réalisé sans positionnement précis avec différents doigts, voire avec la main. Cette rotation évite l'impression d'introduire quelque chose dans son corps et réduit drastiquement la pression vers la peau. Ce geste est réalisé avec un appui fixe qui stabilise le dispositif et l'aiguille, n'est pas sensible à la pression et est naturellement réalisé à vitesse constante. Dans cette position de maintien stable, des pauses en fonction de la perception sont faciles à réaliser.

Une autre contrainte liée aux injections, surtout lorsqu'elles sont réalisées par des non-spécialistes et impliquent des produits visqueux ou pâteux, est de savoir lorsque toute la dose a été injectée. Face à cette incertitude, la tendance est d'appuyer très fort en fin d'injection, entraînant de la douleur par la pression et les mouvements de l'aiguille dans les tissus.

Avec les injections en translation, la visibilité du piston au fond du réservoir de la seringue très proche du point d'injection est en plus toujours difficile, alors qu'avec les injections en rotation telles que celles réalisées avec un dispositif selon l'invention, il est impossible de forcer en pression et l'on peut avoir un point d'arrêt précis et visible de la rotation qui signale la fin de l'injection. De plus, les graduations sont reportées plus haut sur le corps du dispositif où elles sont beaucoup plus faciles à lire qu'à la base, au contact de la peau.

Dans le cas des auto-injecteurs en translation par pression, ces dispositifs exercent une forte pression sur la peau, difficilement compatible et mal tolérée par les patients, surtout pour les enfants et personnes âgées pour qui cela représente une vraie sensation de coup ou de choc.

La présente invention a pour but de proposer de nouveaux dispositifs d'injection qui gardent les propriétés utiles des auto-injecteurs et stylo-injecteurs, mais qui restent simples, petits, légers et robustes et donc facilement transportables comme les dispositifs d'injections classiques de type seringue.

Les nouveaux dispositifs selon l'invention pourront de même servir pour pratiquer des administrations autres que des injections, par exemple topiques, similaires à de la mésothérapie, ou encore intramammaires (en médecine vétérinaire).

La présente invention a ainsi pour objet un dispositif d'injection du type comportant un corps cylindrique renfermant un réservoir contenant un produit à injecter, qui est ouvert à une extrémité sur une aiguille d'injection et qui est fermé à son autre extrémité par un piston monté mobile en translation dans le réservoir sous la commande d'une tige de piston pourvue d'un organe de commande, ce dispositif comportant des moyens de contrôle de la quantité de produit à délivrer, lesdits moyen de contrôle étant disposés sur le corps en une zone se situant hors de la zone de préhension à pleine main du corps, qui est située dans la zone distale du corps, caractérisé en ce que lesdits moyens de contrôle comprennent une tige de piston comportant une partie filetée sur laquelle est vissée une molette de réglage et en ce que le corps comporte deux butées extrêmes, à savoir une butée distale et une butée proximale entre lesquelles la molette de réglage peut être positionnée, ce dispositif permettant à l'utilisateur de sélectionner à l'avance la quantité de produit qui sera injectée à un patient sans avoir, au cours de l'injection, à contrôler la quantité de produit délivré.

La tige de piston pourra comporter une partie filetée sur laquelle sera vissée une molette de réglage, et le corps pourra comporter deux butées extrêmes, à savoir une butée distale et une butée proximale entre lesquelles la molette de réglage pourra être positionnée.

Cette molette de réglage pourra comporter sur sa périphérie des graduations, qui seront visibles au niveau de l'extrémité proximale du corps, et ce dernier pourra comporter au moins un index dont la coopération avec les graduations permettra de déterminer un angle de rotation de ladite molette de réglage et ainsi son déplacement longitudinal par rapport à la partie filetée. La zone de la molette de réglage comportant les graduations pourra dépasser latéralement du corps en au moins une zone.

Par ailleurs la partie proximale du corps pourra recouvrir, au moins en partie, la molette de réglage et pourra comporter au moins une zone transparente au travers de laquelle on pourra apercevoir au moins une des graduations.

La partie proximale du corps pourra former un boîtier cylindrique, notamment ouvert sur l'un de ses côtés, qui sera traversé par une partie filetée de la tige de piston, la molette de réglage étant apte à se déplacer par vissage ou dévissage sur la partie filetée pour aller de la butée proximale à la butée distale ces butées étant par exemple constituées par les deux parois extrêmes du boîtier.

Enfin le chant de la molette de réglage pourra constituer un index dont la coopération avec des graduations portées sur le corps permettra de déterminer un angle de rotation de ladite molette de réglage et ainsi son déplacement longitudinal par rapport à la partie filetée.

Le réservoir du dispositif suivant l'invention pourra être constitué d'une cartouche ou d'une seringue d'injection standards, simples ou à double chambre, dont la tige de piston pourra comporter un élément anti-rotation telle qu'une rainure longitudinale, un méplat sur sa partie filetée en contact avec une partie complémentaire du corps ou du boitier, ou un élément de friction avec la paroi interne dudit réservoir, tel que notamment un joint torique.

Dans un mode de mise en œuvre la partie distale du corps du dispositif d'injection pourra comporter un élément prolongateur, pouvant se terminer par une partie effilée, qui sera traversé par la canule de l'aiguille d'injection, cette canule s'étendant au-delà de la partie distale de l'élément prolongateur d'une longueur égale à la longueur implantable souhaitée de ladite canule.

Suivant l'invention la tige de piston du dispositif d'injection pourra comporter au moins une partie filetée qui sera en prise avec un écrou solidaire du corps, et en ce que l'organe de commande sera en forme de cloche s'étendant vers l'extrémité distale du corps et qui sera apte à recouvrir une partie de ce dernier.

La présente demande décrit une aiguille d'injection comportant une canule solidarisée d'une embase, cette embase pouvant comporter un plateau circulaire axialement centré avec un bossage cylindrique adjacent de même axe, qui sera creusé d'une cavité en communication avec le canal de l'aiguille, cette cavité formant un conduit convergent en allant de sa partie proximale vers sa partie distale.

Dans une variante de mise en œuvre, l'aiguille d'injection comportera une canule solidarisée d'une embase cylindrique formée d'un élément tubulaire comportant un conduit disposé dans le prolongement de celui de la canule, et dont le diamètre interne sera supérieur à celui de cette dernière, le diamètre externe de l'embase pouvant être supérieur à celui de la canule.

Dans un mode de mise en œuvre, le plateau circulaire de l'embase de l'aiguille sera appliqué contre la face distale d'un réservoir, notamment une cartouche, simple ou à double chambre, par des moyens de maintien, notamment des moyens de sertissage. De façon particulièrement intéressante, la liaison entre cette embase et la face distale du réservoir sera réalisée de façon non étanche, de façon que, lorsque l'aiguille se trouve implantée dans une veine, la pression sanguine permette une sortie de l'air et une remontée du sang dans la partie distale de ce réservoir.

Le dispositif d'injection pourra comporter un corps cylindrique renfermant un réservoir notamment constitué d'une cartouche ou d'une seringue, simples ou à double chambre, contenant un produit à injecter, qui sera ouvert à une extrémité sur une aiguille d'injection et qui sera fermé à son autre extrémité par un piston monté mobile en translation dans le réservoir sous la commande d'une tige de piston pourvue d'un organe de commande, le corps comportant des moyens de maintien, éventuellement amovibles, du réservoir, et la longueur du corps étant telle qu'elle laisse dépasser une longueur de canule égale à la longueur que l'on souhaite implanter. La tige de piston pourra comporter au moins une partie filetée qui sera en prise avec un écrou solidaire du corps, l'organe de commande étant en forme de cloche s'étendant vers l'extrémité distale du corps et sera apte à recouvrir une partie de ce dernier.

La présente demande décrit également un dispositif de remplissage dans un réservoir, notamment une cartouche ou une seringue, simples ou à double chambre, d'un produit à injecter, comprenant un récipient de stockage du produit à injecter comportant une buse de remplissage apte à être introduite à l'intérieur du col dudit réservoir. Des moyens d'étanchéité étant prévus entre ledit col et ladite buse, ces moyens d'étanchéité pourront être constitués d'un joint torique disposé dans une rainure circulaire entre le col du réservoir et la buse.

La présente demande décrit également un procédé de remplissage avec un produit de traitement d'un réservoir, notamment une cartouche ou une seringue, simples ou à double chambre, équipé d'un piston et se terminant à son extrémité distale par un col, comprenant les étapes consistant à :

-   positionner le piston à l'extrémité distale du réservoir,
-   introduire dans le col du réservoir une buse d'un récipient de stockage contenant le produit de traitement à injecter,
-   transférer le produit à injecter du récipient de stockage dans le réservoir, le piston de celui-ci étant repoussé par ledit produit à injecter.

La présente demande décrit également un réservoir, notamment une cartouche ou une seringue, simples ou à double chambre, comportant un produit à injecter, un col de réservoir et un piston, dans lequel le produit à injecter s'étend dans le réservoir entre son col et le piston sans espace d'air entre ledit col et ledit piston.

La présente demande décrit également un dispositif de remplissage d'un produit à injecter dans un réservoir, notamment une cartouche ou une seringue, simples ou à double chambre, comportant un piston et à son extrémité distale une aiguille d'injection, comprenant un boîtier récepteur de forme cylindrique qui est percé à chacune de ses extrémités d'un logement cylindrique longitudinal, à savoir un logement réalisé dans sa partie proximale qui est apte à recevoir le réservoir, et un logement réalisé dans sa partie distale qui est apte à recevoir un récipient de remplissage fermé par un septum et comportant des moyens de propulsion du produit de traitement, des moyens de butée étant prévu pour immobiliser le réservoir dans le logement dans une position telle que son aiguille ait perforé le septum et pénétré dans le récipient de remplissage. Préférentiellement le boîtier récepteur sera équipé de moyens aptes à permettre la vision de la position du piston du réservoir lorsque celui-ci sera en place dans son logement et de moyens aptes à mesurer un déplacement du piston.

Le dispositif d'injection suivant l'invention pourra comporter un piston mu par une tige de piston sous l'action d'une tête de commande et, à son extrémité distale, une aiguille d'injection, et l'extrémité proximale de la tige de piston pourra comporter des moyens aptes à coopérer successivement, de façon amovible, avec d'une part une tête de commande de piston à pression et, d'autre part une tête de commande de piston à rotation. Préférentiellement la tige de piston sera de section droite circulaire, et le dispositif d'injection comportera un écrou apte à se fixer sur la partie proximale du réservoir dont le diamètre du trou fileté sera supérieur à celui de la tige de piston et sera inférieur au diamètre interne du réservoir.

Dans un tel dispositif d'injection la tête de commande de piston à pression sera constituée d'un élément cylindrique dont le diamètre sera inférieur à celui du trou fileté de l'écrou, de façon qu'il puisse être reçu dans le réservoir, cet élément cylindrique étant apte à se solidariser de l'extrémité proximale de la tige de piston.

Dans ce même dispositif d'injection la tête de commande de piston à rotation comprendra une vis apte à se visser dans le trou fileté de l'écrou, de façon à venir en appui sur la partie proximale de la tige de piston et être en mesure de la pousser vers l'extrémité distale du réservoir lorsque l'on visse la vis. Cette vis sera préférentiellement solidaire du fond d'un capuchon.

La présente demande décrit également un dispositif de remplissage et de conditionnement d'un ensemble de réservoirs, notamment de cartouches ou de seringues, simples ou à double chambre, comportant un bac récepteur pourvu de moyens aptes à maintenir en position verticale plusieurs rangées de réservoirs, et un couvercle apte à fermer celui-ci, comportant :

- des moyens d'alignement aptes à maintenir le couvercle dans une même position latérale par rapport aux réservoirs,
- des moyens élastiques aptes à repousser le couvercle vers une position d'ouverture,
- des moyens prévus sur la face interne du couvercle du bac récepteur pour maintenir au-dessus de chaque réservoir un piston apte à être reçu dans ce réservoir.

La présente demande décrit également un procédé de remplissage et de conditionnement d'un ensemble de réservoirs, notamment de cartouches ou de seringues, simples ou à double chambre, par un produit dans un dispositif comportant un bac récepteur pourvu de moyens aptes à maintenir en position verticale plusieurs rangées de réservoirs, et un couvercle apte à fermer celui-ci et comportant sur sa face inférieure des pistons maintenus au droit de chaque réservoir, comportant les étapes consistant à :

- remplir les réservoirs contenus dans le bac récepteur avec le produit,
- réaliser un traitement éventuel du produit,
- faire un vide dans le bac récepteur,
- fermer le couvercle de façon à enfoncer les pistons dans les réservoirs,
- casser le vide, de façon à maintenir les pistons dans les réservoirs,
- ouvrir le couvercle.

On décrira ci-après, à titre d'exemple non limitatif, des formes d'exécution de la présente invention, en référence aux dessins annexés sur lesquels :

- la figure 1 est une vue de face en coupe axiale et longitudinale d'un premier mode de mise en œuvre d'un dispositif d'injection suivant l'invention,

- la figure 1a est une vue agrandie de l'extrémité distale du dispositif d'injection représenté sur la figure 1,

- la figure 2 est une vue de dessus du dispositif d'injection représenté sur la figure 1,

- la figure 3 est une vue en coupe du dispositif d'injection représenté sur la figure 1 suivant la ligne AA de celle-ci,

- la figure 4 est une vue de gauche en perspective du dispositif d'injection représenté sur la figure 1,

- la figure 5 est une vue en perspective d'une prise à pleine main du dispositif d'injection représenté sur les figures 1 à 4,

- la figure 6 est une vue de face en coupe axiale et longitudinale d'une variante du mode de mise en œuvre du dispositif d'injection représenté sur les figures 1 à 5,

- la figure 6a est une vue schématique des moyens d'indexation mis en œuvre sur le dispositif d'injection représenté sur les figures 6 et 7,

- la figure 7 est une vue en perspective du dispositif d'injection représenté sur la figure 6,

- la figure 8 est une vue en coupe axiale et longitudinale d'un second mode de mise en œuvre d'un dispositif d'injection suivant l'invention,

- la figure 8a est une vue en coupe longitudinale à échelle agrandie d'un mode de mise en œuvre d'une aiguille d'injection

- la figure 8b est une vue en coupe axiale et longitudinale partielle agrandie d'un mode de réalisation de l'aiguille représentée sur la figure 8a,

- la figure 9 est une vue de dessus du dispositif d'injection représenté sur la figure 8,

- la figure 9a est une vue en perspective d'une prise à pleine main du dispositif d'injection représenté sur les figures 8 et 9,

- la figure 10 est une vue en coupe axiale et longitudinale d'une variante de mise en œuvre d'un dispositif d'injection suivant l'invention,

- la figure 10a est une vue en coupe axiale et longitudinale à échelle agrandie d'une aiguille en mesure d'être utilisée dans le dispositif d'injection représenté sur la figure 10,

- les figures 11 et 12 sont des vues en coupe axiale et longitudinale d'un dispositif d'injection à commande d'injection par rotation respectivement avant et après l'injection,

- la figure 11a est une vue partielle en coupe axiale et longitudinale de la partie distale d'une variante du dispositif d'injection représenté sur la figure 11,

- la figure 13 est une vue en coupe axiale partielle d'un dispositif de remplissage pour cartouche ou seringue, simples ou à double chambre,

- les figures 14 et 15 sont des vues d'un autre dispositif de remplissage pour cartouche ou seringue, simples ou à double chambre, respectivement en coupe axiale et longitudinale et en vue de dessus,

- les figures 16a à 16c sont des vues partielles de l'extrémité proximale d'une seringue apte à réaliser deux modes d'injection possibles, à savoir par pression et par rotation,

- la figure 17 est une vue en coupe transversale d'un dispositif de conditionnement d'une série de cartouches ou de seringues, simples ou à double chambre,

- la figure 18 est une vue en perspective d'un mode de mise en œuvre de l'invention,

- la figure 19 est une vue en coupe axiale et longitudinale du mode de mise en œuvre représenté sur la figure 18,

- la figure 20 est une vue en coupe axiale et longitudinale d'un mode de mise en œuvre d'un dispositif d'injection suivant l'invention représenté en position de repos ou pré injection,

- la figure 20a est une vue agrandie de la zone A du dispositif d'injection tel que représenté sur la figure 20,

- la figure 21 est une vue en perspective du dispositif d'injection représenté sur la figure 20,

- la figure 22 est une vue en coupe axiale et longitudinale du dispositif d'injection de la figure 20 représenté en cours d'injection,

- la figure 22a est une vue agrandie de la zone B du dispositif d'injection tel que représenté sur la figure 22,

- la figure 23 est une vue en coupe axiale et longitudinale du dispositif d'injection des figures 20 et 22 représenté une fois l'injection terminée,

- la figure 23a est une vue agrandie de la zone C du dispositif d'injection tel que représenté sur la figure 23.

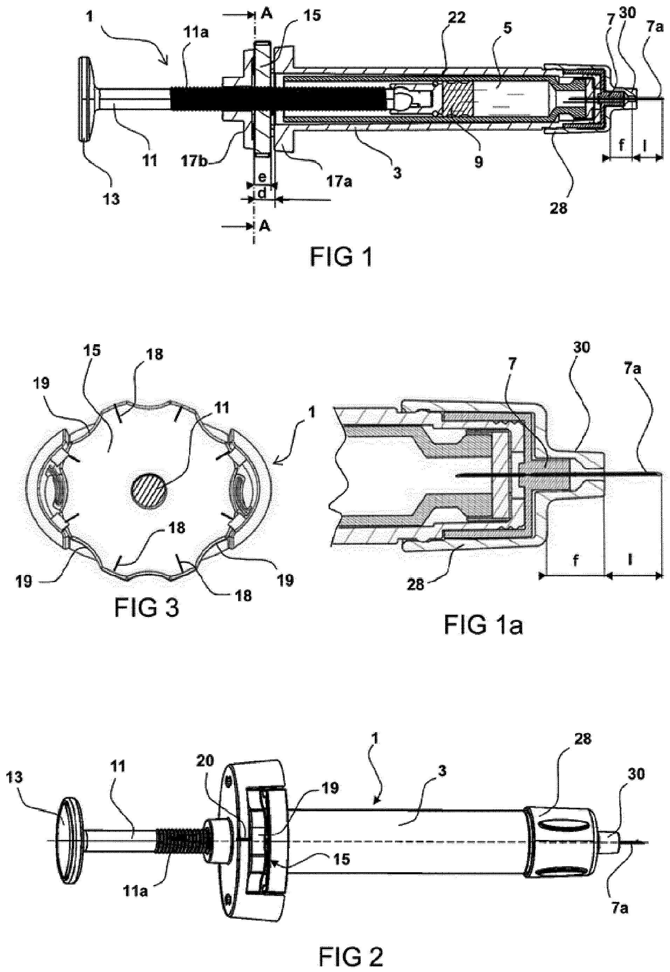

On a représenté sur les figures 1 à 5 un premier mode de mise en œuvre d'un dispositif d'injection suivant l'invention qui est principalement destiné à la réalisation d'injections intradermiques ou sous-cutanées.

Ce dispositif d'injection 1, dont la réalisation se présente sous la forme semblable à celle d'une seringue, comprend un corps tubulaire 3 à l'intérieur duquel est disposé un réservoir 5 contenant un produit à injecter et qui se termine par une aiguille d'injection 7 pourvue d'une canule 7a. De façon connue, un piston 9 est monté coulissant à l'intérieur du réservoir 5 sous l'action d'une tige de piston 11 dont l'extrémité externe, dite proximale dans le présent texte, comporte une tête de commande d'injection 13.

De façon à assurer un bon contact du piston 9 avec le réservoir 5 et pour obtenir une bonne précision de dosage, ainsi que pour résister aux pressions opérées, par exemple lors de l'injection de produits visqueux ou pâteux, on pourra avantageusement utiliser suivant l'invention, un piston plein, de type classique ainsi qu'utilisé par exemple dans les cartouches dentaires ou dans certains stylo-injecteurs.

La tige de piston 11 comporte une partie filetée 11a sur laquelle est vissé un écrou formant une molette de réglage 15, d'épaisseur e, qui est apte à se déplacer longitudinalement entre deux butées du corps tubulaire 3, respectivement une butée distale 17a et une butée proximale 17b qui sont écartées d'une distance $\underline{d}$ qui est fonction de la quantité maximale de produit que l'on souhaite pouvoir injecter, ainsi qu'exposé ci-après.

Dans une variante de mise en œuvre de l'invention, la tige de piston 11 sera pourvue sur sa périphérie d'un élément de friction constitué d'un joint torique 22 disposé dans une rainure de celle-ci qui sera appliqué contre les parois du réservoir 5 de façon à créer une force de friction permettant d'éviter la rotation de la tige de piston 11 lors de l'injection ou lorsque le dispositif n'est pas utilisé ou lorsque la molette de réglage 15 est actionnée.

Ce joint torique 22 pourra assurer cet effort de friction sans créer de barrière étanche, par exemple en prévoyant des ouvertures, non représentées sur le dessin, réalisées dans ladite rainure. De cette manière la tige de piston 11 peut être introduite dans le réservoir 5 jusqu'au contact avec le piston 9 sans emprisonner d'air entre son extrémité distale et le piston.

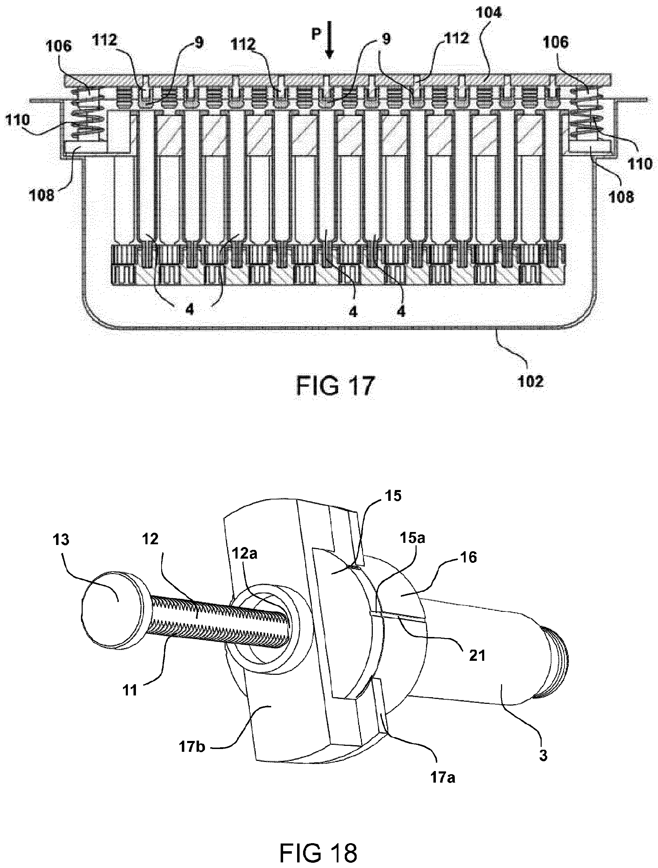

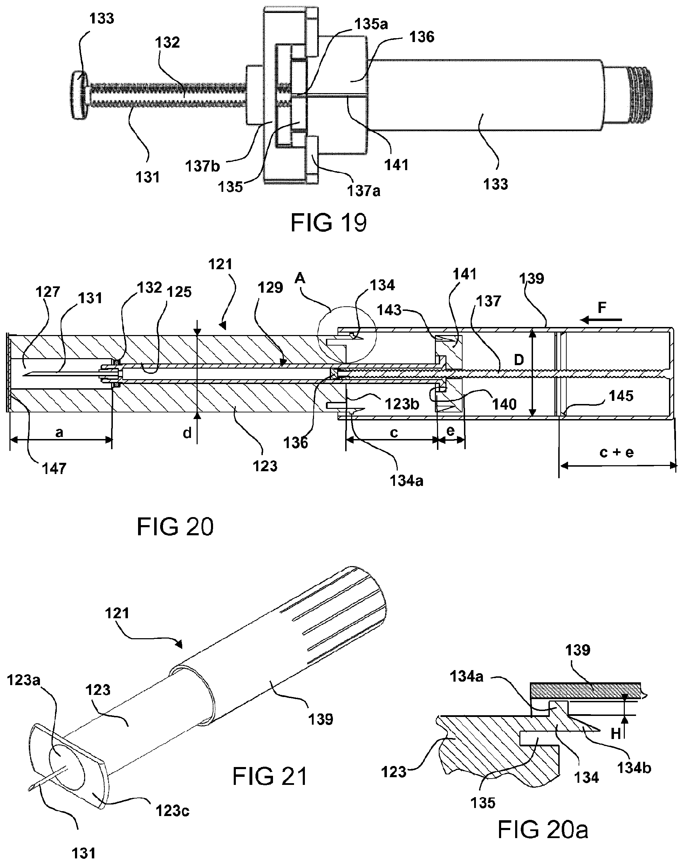

Dans une autre variante de mise en œuvre de l'invention, qui est représentée sur les figures 18 et 19, la tige de piston 11 comportera un méplat longitudinal 12 dont la fonction sera d'empêcher sa rotation lors de son déplacement longitudinal lors de l'injection. A cet effet au moins l'une des deux butées 17a, 17b comportera une partie plane 12a contre laquelle le méplat 12 de la tige de piston 11 sera en appui. De cette façon, la tige de piston 11 ne pourra pas entrer en rotation, et ne pourra se déplacer qu'en translation lors de l'injection.

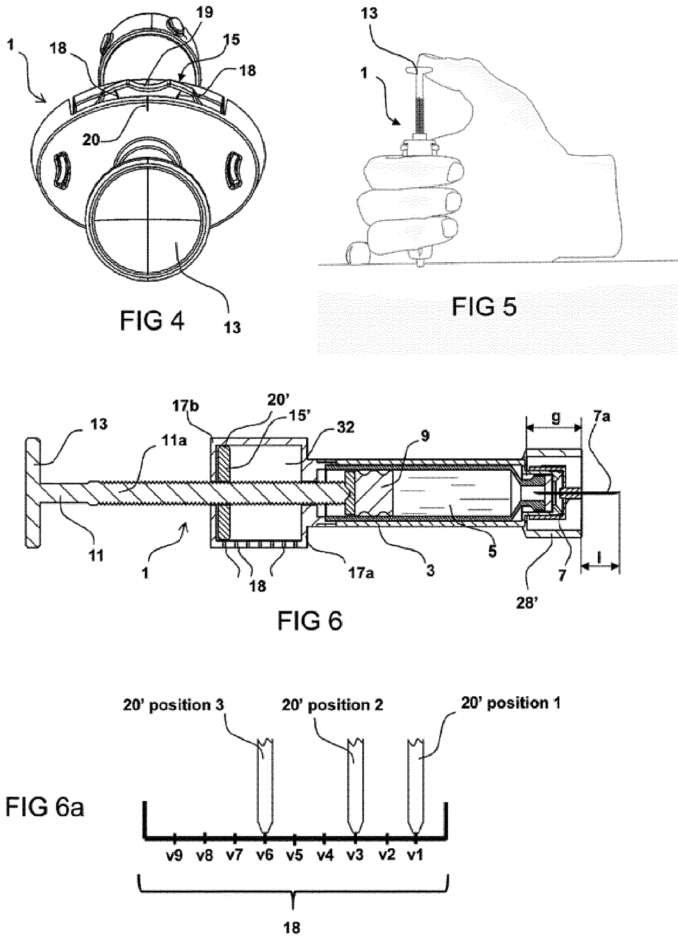

Ainsi que représenté sur les figures 3 et 4, la molette de réglage 15 se présente, dans le présent exemple de mise en œuvre, sous la forme d'un disque comportant sur sa périphérie des cannelures 19 destinées à améliorer la préhension de la molette 15 par l'utilisateur. La face proximale de la molette de réglage 15 comporte sur sa périphérie des graduations 18 qui sont réparties, notamment de façon régulière, sur la périphérie de celle-ci et qui sont destinées à coïncider avec un index 20 formé sur la butée proximale 17b du corps 3. Dans une variante de cet exemple de mise en œuvre, on pourrait également réaliser la butée proximale 17b en un matériau transparent de façon à permettre à l'utilisateur de lire les graduations 18 au travers de cette dernière.

Dans ces conditions, après que l'utilisateur a effectué le « priming » c'est-à-dire l'opération précédant l'injection par laquelle il évacue l'air contenu dans le réservoir 5 du dispositif d'injection, la molette 15 se trouve en contact avec la butée distale 17a. L'utilisateur tourne alors cette molette pour la positionner sur la graduation choisie ce qui a pour effet de la reculer de la distance nécessaire à l'éjection de la quantité de produit souhaité ainsi que représenté sur la figure 1.

A titre d'exemple, si le pas de la partie filetée 11a est égal à $p$, et si la molette 15 comporte N graduations et que le diamètre du réservoir 5 est de D mm on comprend que le volume injecté pour une rotation de la molette de n divisions sera égal à :

$$(p \times n)/N \times \pi . D2/4$$

Ainsi, pour un pas de vis p=0,2mm, un nombre de graduations N = 20 et un diamètre du réservoir D = 8mm, chaque graduation correspondra à un volume injecté de 0,5 mm3.

Le dispositif d'injection selon l'invention peut être équipé, comme représenté sur les figures 18 et 19, d'une bague de remise à zéro 16. Cette bague permettra à l'utilisateur, s'il le souhaite, de sélectionner une dose différente de produit à injecter à chaque administration. A cet effet la bague de remise à zéro 16 est montée mobile en rotation sur le corps 3 et comporte un index repère 21 qui constitue un point de départ pour la sélection d'une dose.

En effet, une fois qu'une première dose est sélectionnée et administrée, il faut à l'utilisateur un point de repère pour réaliser une remise à zéro afin de pouvoir sélectionner une deuxième, troisième, etc... dose. Chaque dose pouvant varier, le zéro de la molette de réglage 15 qui est matérialisé par un index 15a, se retrouve après chaque administration à une position différente. Suivant l'invention, pour régler une nouvelle dose il suffira à l'utilisateur de positionner l'index 21 de la bague de remise à zéro 16 en face de l'index 15a de la molette de réglage, puis de faire tourner celle-ci d'un angle par rapport à l'index 21 correspondant à la nouvelle dose souhaitée.

La présente invention est tout d'abord particulièrement intéressante en ce qu'elle permet à l'utilisateur de sélectionner à l'avance la quantité de produit qui sera injectée à un patient, si bien qu'il n'a pas à se préoccuper, au cours de l'injection, du contrôle de la quantité de produit délivré.

De plus, on constate qu'elle permet d'injecter, de façon particulièrement précise, des quantités de produit extrêmement faibles qui ne pourraient l'être au moyen des dispositifs de l'état antérieur de la technique. On constate aussi que l'injection de ces micro-volumes, par exemples inférieurs à 0.05mL, n'est pas douloureuse pour le patient, ce qui permet par exemple de répéter les injections.

Ces doses de micro-volumes (de quelques microlitres) sont particulièrement utiles dans de nombreux domaines thérapeutiques, tels que pédiatrique, vétérinaire (petits animaux), dermatologie (injection intradermique de produits contre l'acné, les verrues et autres maladies de la peau), les vaccins ou certaines applications de toxine botuliques, ou encore en cosmétique, pour des applications de type « mésothérapie ». Dans le cas des applications cosmétiques, le dispositif pourra être équipé d'une ou plusieurs aiguilles très fines, par exemple de 0.1mm de diamètre et de 0.2mm de longueur, voire ne pas être équipé d'une aiguille mais d'un embout dispenseur. Par ailleurs, la présente invention permet d'utiliser le présent dispositif d'injection non plus comme une seringue de type classique qui est tenue entre deux doigts et le pouce de l'utilisateur, à savoir en prise interdigitale latéro-latérale, mais par exemple en prise digito-palmaire, c'est-à-dire à pleine main sur le corps 3, le pouce de l'utilisateur étant positionné sur la tête 13 de la tige de piston 11 ainsi que représenté sur la figure 5.

Une telle prise en main permet notamment une utilisation facile pour une auto-administration de volumes contrôlés de produit. Ce type de prise en main permet aussi d'envisager facilement une présentation multi-doses donnant la possibilité de pratiquer l'injection de ces volumes contrôlés de manière répétitive, pour être utilisé par exemple en mésothérapie ou pour l'application de produits de type « fillers » en faisant varier les sites d'injection.

La demanderesse a également constaté que cette prise en main permettait d'exercer facilement et confortablement sur le piston 9 des forces d'injection beaucoup plus importantes que celles que l'on était en mesure d'exercer avec les dispositifs d'injection conventionnels, et avec une grande précision de dosage, et ce sans que l'utilisateur ait à subir les mouvements parasites habituels des injections classiques. Une telle prise en main permet également, par voie de conséquence, d'utiliser, pour un même produit à injecter, des aiguilles dont la canule est beaucoup plus fine que celles utilisées précédemment.

En effet la présente invention permet de séparer deux fonctions qui, jusqu'à présent se trouvaient confondues, à savoir d'une part la fonction de préhension et d'autre part la fonction d'injection. Ainsi, suivant l'invention, la préhension du dispositif d'injection est fixe pendant toute la durée de l'injection puisqu'elle est assurée « à pleine main » c'est-à-dire par les quatre doigts de la main de l'utilisateur sauf le pouce et que l'injection est assurée quant à elle par le pouce de celui-ci. Dans les dispositifs d'injection de type classique au contraire la préhension, qui est assurée par l'index et le majeur aidés du pouce, varie tout au long de l'injection puisque le pouce qui se déplace, constitue l'un des éléments de maintien du dispositif d'injection.

Or la demanderesse a constaté que cette séparation des fonctions permet d'une part d'exercer plus facilement des forces d'injection plus importantes ce qui autorise l'injection de produits plus visqueux tels que, par exemple des « fillers » biopolymériques, tels que d'acide hyaluronique, de collagène ou d'élastine, où même d'acide polylactique (PLA), ou de diminuer la taille de l'aiguille utilisée pour l'injection d'un produit donné et, d'autre part, de ne pas exercer de pression excessive à la surface de la peau du patient pendant l'injection.

On comprend de ce qui précède que cette prise en main est rendue possible par le fait que le contrôle de la quantité du produit à injecter s'effectue en dehors de la zone de préhension à pleine main du dispositif d'injection, c'est-à-dire, dans cet exemple, dans la zone distale du dispositif d'injection.

L'efficacité de la prise à pleine main permise par la présente invention est encore améliorée, dans une variante de mise en œuvre de l'invention, en dotant la partie distale du dispositif d'injection d'un élément prolongateur 28 permettant de régler la profondeur d'injection, ainsi que représenté sur les figures 1, 1a et 2, qui sera apte à se fixer, par exemple par vissage ou clipsage, sur l'extrémité distale du corps 3.

Cet élément prolongateur 28 se termine vers l'avant par une partie effilée 30 qui est traversée par la canule 7a d'une aiguille 7 et qui est du type commercial connu existant pour les stylo-injecteurs. Cet élément prolongateur 28 est d'une longueur et d'un diamètre réduits, ce diamètre étant très inférieur au diamètre du corps 3, de façon à permettre à l'utilisateur d'une part une visée précise du point d'injection et d'autre part de lui permettre de contrôler la profondeur souhaitée d'implantation de la canule de l'aiguille. Ainsi, au cours de l'injection, l'utilisateur amènera en appui l'extrémité de la partie effilée 30 en contact avec la peau du patient, si bien que la longueur d'implantation de la canule 7a de l'aiguille 7 sera égale à la distance 1 dont elle dépasse de ladite partie effilée 30.

Pour régler la longueur d'implantation on pourra ainsi soit jouer sur la longueur f de la partie effilée 30 soit sur la longueur l de la canule de l'aiguille 7.

Ainsi, l'utilisateur pourra disposer d'une série d'éléments prolongateurs 28 dont les longueurs f de leur partie effilée 30 seront différentes et qui lui permettront de régler la longueur l implantable de la canule 7a en fonction de la profondeur de l'injection souhaitée.

Par exemple, dans les cas des applications de produits cosmétiques, il est possible de régler la longueur de l'élément prolongateur pour que la longueur implantée soit tellement faible que la canule même de l'aiguille ne puisse pas pénétrer dans le derme, et que la surface de la peau, ou stratum corneum, ne soit pénétré que par la pointe du biseau d'une épaisseur par exemple de 0.10mm. Dans ce cas très particulier, la pénétration du produit cosmétique dans la peau sera améliorée grâce à ces microlésions créées par la pointe de la canule de l'aiguille, comme par exemple lors de traitements par mésothérapie, mais le produit ne sera pas injecté, mais plutôt déposé en surface sur les microlésions.

Dans un autre mode de mise en œuvre du dispositif d'injection l'aiguille peut être fixée directement dans l'élément prolongateur 28 de façon à constituer une seule pièce, ce qui permettra à l'utilisateur de disposer de divers éléments prolongateurs possédant des longueurs l de canules différentes qu'il choisira en fonction de la profondeur de l'injection qu'il souhaite réaliser.

On notera que cet élément prolongateur 28 assure ainsi trois fonctions, à savoir une fonction de réglage de la longueur implantée de la canule de l'aiguille dans le corps du patient, une fonction de stabilisation du dispositif au cours de l'opération d'injection puisque la base distale de sa partie effilée 30 reste en appui au contact du corps du patient, et une fonction de visée fine du point d'injection qui permet de positionner avec précision la pointe de l'aiguille à l'endroit souhaité. En effet, le dispositif étant amené à être utilisé avec des aiguilles extrêmement fines (29G ou plus), il est difficile de viser le site d'injection, l'aiguille exposée étant trop fine et donc très peu visible.

Le dispositif d'injection suivant l'invention permet ainsi de réaliser une injection intradermique avec précision, par exemple dans une ride à une profondeur maximale déterminée et reproductible. On peut également adapter ladite longueur en fonction d'autres objectifs ou sites d'injections précis, tels que par exemple pour des vaccins ou pour l'injection intradermique de peptides et protéines, des produits pour traiter le diabète, comme par exemple l'insuline ou les GLP-1 analogues, plus particulièrement d'insuline préprandiale, qui présente une rapidité d'action supérieure lorsque injecté en intradermique.

Ce dispositif d'injection selon l'invention est particulièrement adapté dans le cadre des administrations en pédiatrie, ou les doses de produits thérapeutique sont particulièrement basses, menant à des volumes réduits. Il sera par exemple possible d'utiliser un produit commercial, et sans changer de concentration, simplement, grâce à un dispositif selon l'invention, administrer une petite dose de ce même produit avec une grande précision. Ceci permet de garder le même produit, déjà enregistré, et sans passer par toutes les étapes de reformulation et de certaines phases d'études cliniques, longues et onéreuses, de changer d'aire thérapeutique simplement grâce à ce nouveau dispositif spécifique.

Dans un mode de réalisation particulier du dispositif d'injection on fera appel à une partie effilée 30 qui recouvre entièrement l'aiguille 7, et qui peut prendre différentes formes et dimensions, de façon à constituer un dispositif d'administration topique d'un produit particulièrement utile notamment dans le domaine de l'ophtalmologie, car permettant une meilleure stabilité lors de l'administration, sans toucher la surface de l'œil.

Dans une variante de l'invention et ainsi que représenté sur la figure 1, le réservoir peut être constitué d'une cartouche de type standard telle que par exemple les cartouches dentaires ou des cartouches à double chambre pour reconstitution extemporanée, de volume standard pouvant varier entre 1ml et 3ml et dont la fabrication, le remplissage et la fermeture par un élément de fermeture de type septum scellé par une bague, présente l'avantage de pouvoir être réalisé sur des lignes de productions existantes à un coût raisonnable.

La présente invention permet de constituer un dispositif d'injection qui est de type multi-doses, c'est-à-dire qu'il peut, à partir d'un réservoir ou d'une cartouche, même à double chambre, délivrer plusieurs injections d'un volume réglable par des positionnements successifs de la molette de réglage 15, sans avoir à subir entre chaque traitement, un rechargement en produit. Ce dispositif multidose est particulièrement adapté à l'injection de très petits volumes dans des conditions précises, telles qu'en injection intradermique. On a de plus constaté qu'en raison de la taille réduite des aiguilles et du volume injecté, par exemple inférieur à 0.05mL, il était possible de réaliser des injections non-douloureuses, permettant donc de multiplier les points d'injection dans la même zone d'administration. Ce genre de dispositif selon l'invention est par exemple particulièrement adapté à l'administration de produits contre l'hyperhidrose dans la paume des mains ou dans la plante des pieds, tels que la toxine botulinique, où il est nécessaire de répéter de nombreuses fois l'injection de petites doses de produits dans le derme du patient. Il peut aussi servir à injecter en intradermique dans la peau du crâne de patients souffrant d'alopécie, des produits thérapeutiques qui auront une efficacité améliorée par rapport à une injection classique si l'on cible les zones intradermiques auxquelles ce type de toxine à une efficacité.

Un tel dispositif d'injection se prête particulièrement bien à une réalisation d'un dispositif de type jetable, notamment lorsque son réservoir est constitué d'une cartouche, simple ou à double chambre, de type standard qui sera alors scellée de fabrication dans le corps 3.

Le dispositif d'injection selon l'invention peut être préchargé du produit à injecter. Il peut aussi être vide avec le piston 9 par exemple en contact avec le col de la cartouche et être chargé au moment de l'utilisation, par exemple avec un dispositif de remplissage décrit ci-après pour remplir les seringues qui correspondent à un autre exemple de mise en œuvre de l'invention. On pourra alors l'utiliser avec une toxine botulique préalablement reconstituée à partir de sa présentation initiale. Le contenu de ce flacon sera alors transféré dans le dispositif, par exemple à l'aide d'une seringue.

Un tel dispositif peut aussi utiliser une cartouche à double chambre contenant un produit lyophilisé et son milieu de reconstitution en amenant le piston intermédiaire au niveau d'une zone de by-pass pour être reconstitué directement dans le dispositif selon l'invention. Dans ce mode de mise en œuvre, la tige de piston 11 comportera une partie distale lisse pour procéder à la reconstitution, suivie d'une partie proximale à vis pour réaliser l'administration telle que décrite précédemment. Pour éviter une possible rotation, cette tige de piston pourra aussi comprendre un méplat servant à guider la reconstitution.

Il est alors possible de prévoir un mécanisme de blocage, par exemple par un système de clé, de la partie lisse de la tige, correspondant à la position du premier piston (distal) au niveau de l'emplacement sur la zone latérale de by-pass. L'utilisateur doit alors retirer ce système de blocage pour finir la translation du second piston (proximal) jusqu'au contact du premier pour pouvoir procéder à l'administration du produit reconstitué. Le fait de devoir retirer le système de blocage oblige l'utilisateur à stopper la course du piston et laisse donc le temps d'une bonne réhydratation une fois les deux produits mis en contact. Le mécanisme de blocage peut aussi être, dans une autre variante, constitué de butées à franchir lorsque la vis est équipée de méplats : une première butée pour enclencher le déplacement des pistons, et une seconde butée pour faire franchir la zone de by-pass au piston distal, avant de procéder à l'administration du produit reconstitué tel que décrit précédemment.

Finalement, ce dispositif peut comporter un système empêchant de sélectionner une dose plus grande que celle restant dans le réservoir, la tête de vis peut venir se clipper sous le boitier du dessus et au contact de la couronne quand la cartouche est vide. On ne peut alors plus tourner la couronne pour sélectionner une dose, et le dispositif se retrouve verrouillé une fois vidé. On peut aussi prévoir un arrêt précis à la rotation de la couronne sous forme d'un téton ou pièce en butée placée sur le boitier, qui empêchera la molette de réglage 15 de tourner au-dessus d'une certaine longueur de piston correspondant à la dose maximale de produit contenue dans le réservoir pouvant être administrée. Ledit téton peut circuler jusqu'au bout d'une rainure de la face supérieure de la couronne qui fixe alors ce maximum. Il n'est libéré ensuite que lorsque la couronne redescend suite à l'administration de la dose sélectionnée.

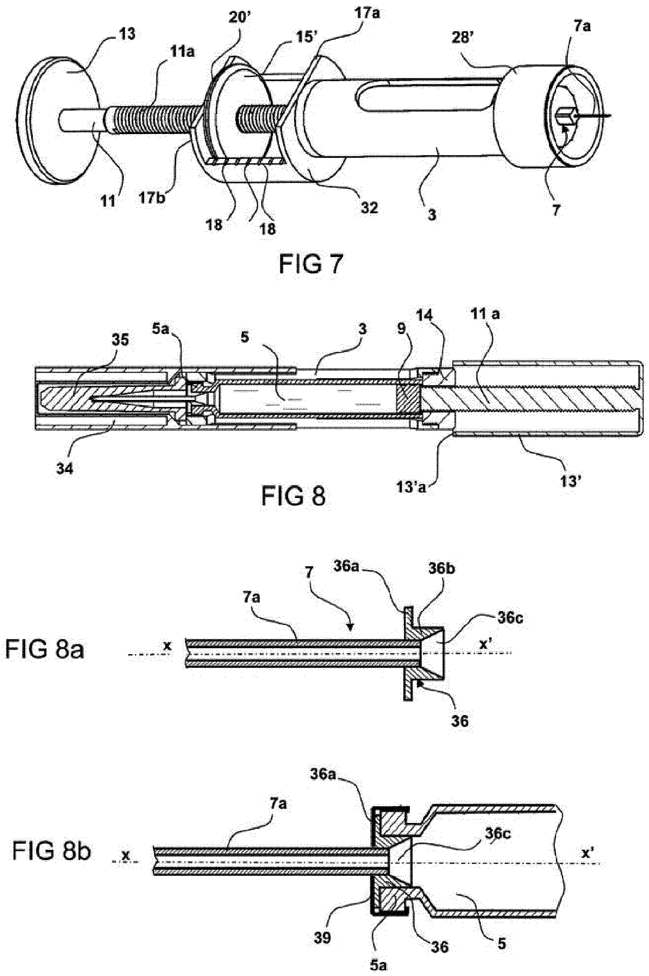

On a représenté sur les figures 6 et 7 une variante du présent mode de mise en œuvre de l'invention qui est particulièrement adaptée à l'injection soit de doses de produit plus importantes, soit à la mise en œuvre de multi-injections.

Dans ce dispositif la partie proximale du corps 3 forme un boîtier cylindrique 32, par exemple ouvert sur l'un de ses côtés, qui est traversé par la partie filetée 11a d'une tige de piston 11. Dans cette variante, la molette de réglage 15' a la forme d'un disque et peut se déplacer, ainsi que dans l'exemple précédent des figures 1 à 5, par vissage ou dévissage sur la partie filetée 11a pour aller d'une butée proximale 17b à une butée distale 17a respectivement constituées par les deux parois extrêmes du boîtier 32.

L'index est ici constitué par le chant 20' de la molette de réglage 15' et des graduations 18 sont portées sur le corps du boîtier 32.

Par ailleurs, l'extrémité distale est pourvue d'un élément prolongateur 28' constitué d'une bague cylindrique encliquetée sur le corps 3 et possédant une longueur g telle qu'il laisse dépasser la canule 7a de l'aiguille 7 d'une longueur 1 égale à l'implantation souhaitée.

On comprend que, ainsi que représenté schématiquement sur la figure 6a, en positionnant la molette 15' en allant de droite à gauche sur le dessin, dans les positions 1, 2 et 3 on est en mesure de réaliser des injections respectives de volume V1 en position 1, de volume V3-V1 en position 2, et de volume V6-V3 en position 3.

Ce dispositif d'injection permet ainsi d'effectuer, de façon préréglée, des injections de doses précises en des zones données précises du corps d'un patient, rendant ainsi aisées les injections intradermiques ou souscutanées, si bien que celles-ci peuvent être pratiquées par un utilisateur peu expérimenté ou ayant des problèmes de dextérité, ou encore par le patient lui-même en auto-injection.

On a représenté sur les figures 8 à 9 un autre mode de mise en œuvre d'un dispositif d'injection suivant la présente invention qui comprend essentiellement un corps tubulaire 3 à l'intérieur duquel est logé un réservoir 5 dans lequel un piston 9, du type de celui décrit précédemment, est monté mobile longitudinalement, sous l'action d'une tige de piston 11a constituée d'une vis. A son extrémité distale cette tige de piston 11a est solidarisée d'un piston 9 et s'étend au travers d'un écrou 14 solidaire du corps 3 pour se terminer à son extrémité proximale par une tête 13' en forme de cloche. Cette dernière recouvre la tige de piston 11a ainsi qu'une partie du corps 3.

On comprend dans ces conditions que la rotation de la tête 13' commande le déplacement longitudinal du piston 9 dans le réservoir 5 et l'expulsion d'un volume donné de produit de traitement par l'aiguille d'injection 7.

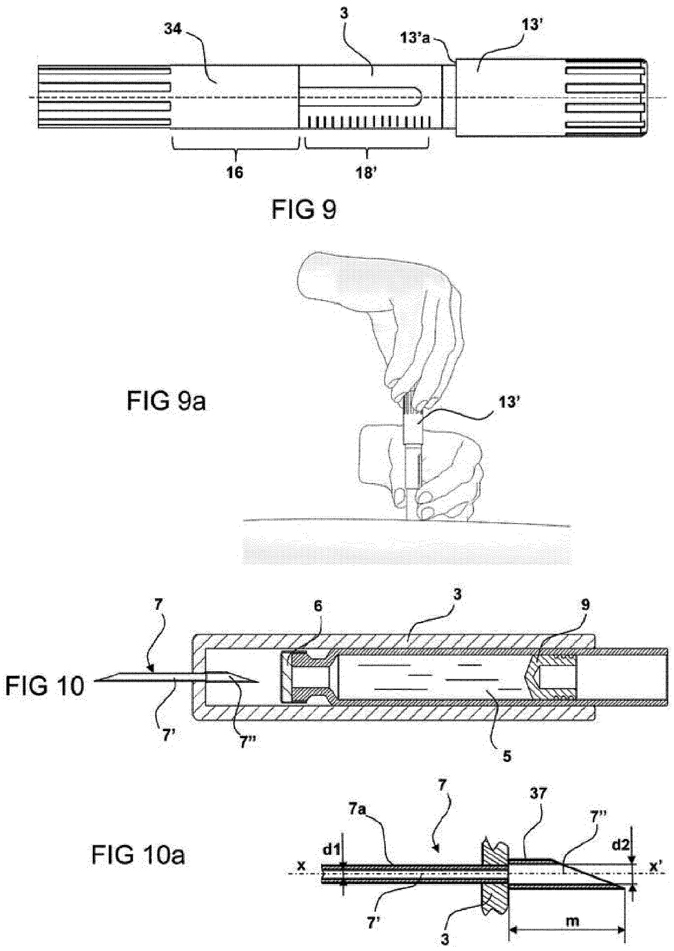

Ce volume injecté est contrôlé au moyen de graduations 18' disposées sur le corps 3 et l'index peut ici être constitués par le bord distal 13'a de la tête 13' ainsi que représenté sur la figure 9. Ce mode de réalisation est particulièrement intéressant en ce qu'il permet de réaliser la lecture sur la partie postérieure ou proximale du corps 3, la partie antérieure ou distale de celui-ci étant attribuée à la prise à pleine main dans une zone 16 dite ciaprès « zone distale de maintien ».

Bien entendu, suivant l'invention, on peut faire appel à tout autre système de mesure dès lors que celui-ci se situe hors de la zone dans laquelle l'utilisateur tient à pleine main le corps 3 c'est-à-dire dans la zone distale de maintien 16. Un tel système de mesure peut également être constitué par des index longitudinaux respectivement disposés sur le corps 3 et sur la tête 13', qui pourront coïncider à chaque tour donné à la vis 11'.

Dans le présent exemple de mise en œuvre, et ainsi que représenté sur les figures 8, 8a et 8b l'aiguille d'injection 7 comporte une embase 36 sur laquelle est fixée sa canule 7a, notamment par collage, par emmanchement à force, par soudure, par exemple par soudure à faisceau d'électrons. Cette embase 36 est formée de première part d'une collerette 36a ayant la forme et les dimensions du septum qui ferme habituellement les réservoirs, notamment constitués par des cartouches standard. La face proximale de cette collerette s'étend vers l'intérieur du col 5a de la cartouche par un bossage 36b. Ainsi que représenté sur les figures 8, 8a et 8b, ce bossage est creusé d'une cavité 36c de forme tronconique de façon qu'elle forme un convergent en allant de sa partie proximale vers sa partie distale.

Le bossage 36b a un diamètre tel qu'il lui permet de se loger dans le col 5a de la cartouche et l'embase 36a est fixée au col 5a de celle-ci par des moyens de sertissage tels qu'une bague 39, ainsi que l'est le septum sur les cartouches classiques.

On constate qu'un tel mode de mise en œuvre procure deux fonctions qui ne se retrouvent pas dans l'état antérieur de la technique.

De première part il permet de réduire autant que faire se peut la longueur inutile de la canule 7a en permettant que l'extrémité proximale de cette dernière se situe quasiment au niveau du col de sortie 5a de la cartouche 5.

De seconde part le convergent qui est formé par la cavité 36c favorise l'écoulement du produit à injecter.

Ainsi ces deux fonctions nouvelles générées par un tel mode de mise en œuvre coopèrent pour diminuer l'importance de la perte de charge imposée au produit injecté au cours de l'opération d'injection, ce qui permet, pour un diamètre interne de canule 7a donné, de diminuer la force nécessaire à l'injection de celui-ci, soit pour une force d'injection donnée de diminuer le diamètre interne de la canule 7a.

Bien entendu, suivant l'invention, on pourrait mettre en œuvre les mêmes fonctions avec des réservoirs autres que des cartouches standard tels que, par exemple, des seringues ou d'autres containers classiques en verre, en matière plastique ou en tout autre matériau, quels que soient d'ailleurs leurs volumes respectifs.

Ce dispositif permet d'injecter des produits beaucoup plus visqueux voire pâteux que de nombreux dispositifs commerciaux, tout en utilisant des containers classiques. Injecter de tels produits visqueux ou semi-solides permet de réduire le volume d'administration pour certains malades requérant de hautes doses thérapeutiques, en oncologie par exemple, avec des anticorps monoclonaux. Cette viscosité élevée permet d'augmenter la concentration en principe actif et, par exemple, d'aller au-delà de leur solubilité, vers des solutions sous-cutanées dans lesquelles les molécules de ces principes actifs pourraient avoir des arrangements spécifiques, contribuant à leur concentration, à leur stabilité et à leur injectabilité, en réduisant par exemple les contraintes de cisaillement appliquées lors de leur administration. Ce dispositif facilite ainsi les injections particulières, comme intra-articulaires, ou intravitréales, ou encore les colles biologiques ou les fillers pour les rides, avec plus de force et de précision avec des aiguilles plus fines, même pour des produits plus visqueux ayant une plus longue durée d'action.

Le dispositif selon l'invention permet de réguler la vitesse d'administration en fonction du ressenti du patient (douleur), et d'effectuer des pauses pendant l'administration. Pour une injection, du fait de sa grande stabilité au site d'injection, il est possible d'attendre un peu avant de poursuivre l'administration sans effectuer de mouvements parasite de l'aiguille implantée, ce qui est actuellement difficile avec les dispositifs d'injection actuels en translation.

Dans le mode de mise en œuvre représenté sur la figure 8 le corps 3 est pourvu d'un capuchon 34 qui vient s'ajuster sur celui-ci et qui permet de recouvrir l'aiguille 7. Ce capuchon comporte un insert interne 35, notamment en caoutchouc ou en butyle qui, en position fermée, vient en appui sur le biseau de la canule 7a de l'aiguille 7 de façon à assurer l'étanchéité de la cartouche 5.

Bien entendu, le capuchon 34 pourra ne pas comporter de systèmes venant en contact avec la canule 7a de l'aiguille 7, par exemple si le produit à injecter est d'une viscosité qui ne permet pas, sans pression, son écoulement à travers la canule. Ceci permet d'obtenir une aiguille prémontée sur le réservoir qui n'a souffert d'aucun contact avant son implantation.

Ce capuchon, de par son ergonomie, permettra de reboucher le dispositif selon l'invention à une seule main en le laissant posé sur la table, évitant ainsi le risque de blessure par piqure d'aiguille. De même, il ne pourra pas être activé par un enfant.

Dans un autre mode de mise en œuvre de l'invention, le réservoir peut être une seringue standard pré-remplie, par exemple de type Hypak de 0.5mL, de diamètre plus petit que celui des cartouches standard, comportant une extrémité luer distale fermée par un bouchon en butyle standard, et des ailettes de maintien à son extrémité proximale.

Bien que possédant un diamètre interne inférieur à celui des cartouches standards, plus adapté à l'administration de produits visqueux, les seringues de 0.5mL présentent le désavantage de ne pas être luer-lock à leur extrémité distale, ou de ne pas présenter de maintien spécifique lors de l'injection de produits visqueux.

Grâce au dispositif selon l'invention, il est possible d'obtenir un montage luer-lock classique mais plus résistant, sans risque de rotation ou déconnection de l'aiguille, donc plus adapté aux produits visqueux.

La seringue est introduite dans le corps 3 qui vient au contact de la peau lors de l'injection. Dans cette mise en œuvre spécifique, la partie distale du corps 3 est terminée par un embout de type luer-lock mâle, sans cône luer ; le cône luer de la seringue de 0.5mL ainsi que son bouchon spécifique en butyl dépassent par cette extrémité distale. Les ailettes en verre de l'extrémité proximale de la seringue peuvent circuler dans deux encoches aménagées de part et d'autre de la partie proximale du corps 3, jusqu'à une certaine distance l où les ailettes se retrouvent en butée distale dans des encoches dans une position basse avant utilisation. À ce moment, le cône luer de l'aiguille est exposée par la partie distale du corps 3, et il est possible de retirer le bouchon butyle pour le remplacer par une aiguille standard. La fixation de l'aiguille en rotation dans le luer-lock entraîne le déplacement de la seringue à l'intérieur du corps 3 en translation dans les encoches jusqu'à une position haute de verrouillage, dans laquelle les ailettes se retrouvent en butée proximale. Le déplacement des ailettes dans le corps 3 sert de contrôle visuel pour s'assurer du montage de l'aiguille. Dans cet arrangement nouveau, les ailettes de la seringue en verre, fragiles, ne supportent plus toute la force de pression lors de l'injection, répartie sur la partie distale de la seringue en verre, depuis la surface externe du cône luer à travers l'embase plastique et la partie luer-lock du corps plastique au niveau distal, jusqu'à l'extrémité proximale du corps 3.

Ce nouveau montage plus résistant autour de l'extrémité luer-lock va permettre d'exercer des forces d'injections plus élevées. Il est aussi possible d'utiliser ce nouveau montage luer-lock dans une version de seringue classique, avec un corps terminé dans sa partie proximale par un appui-doigt et une tige de piston standards.

Dans une autre mise en œuvre d'un dispositif selon l'invention autour d'une seringue de 0,5mL, ce dispositif pourra avoir les mêmes fonctions que celles décrites précédemment, mais dans ce cas particulier, le dispositif sera préférentiellement associé à la seringue préremplie par clippage latéral ; ceci permet d'ajouter une pièce spécifique qui puisse venir se clipper sur le cône de l'embase de l'aiguille pour contrôler et assurer sa bonne insertion et son maintien sur l'extrémité luer de la seringue. Cette partie clippée du corps 3 peut comporter les autres éléments selon l'invention aux fonctions déjà décrites, telles que l'appui sur la peau à son extrémité distale ou la possible présence d'un système de protection collant.

Cette pièce spécifique du dispositif qui vient se clipper latéralement sur l'embase assure une connexion suffisante entre la seringue et l'aiguille pour permettre l'injection de produits visqueux. Dans certaines applications, le dispositif selon l'invention pourra aussi être prolongé à son extrémité proximale par un appui-doigt associé à une tige de piston pour permettre son utilisation comme seringue classique.

Toujours autour d'un réservoir seringue préremplie de 0,5mL, il est possible de prévoir une version à aiguille fixe, comparable à celle envisagée pour des cartouches. Dans ce cas particulier, une aiguille à embase métallique peut être introduite par l'ouverture proximale du réservoir. Cette embase à une forme adaptée au diamètre interne du réservoir pour venir en butée à l'intérieur de la seringue, du côté de l'extrémité luer. La canule de cette aiguille dépasse alors par l'ouverture de cette extrémité ; il est possible de boucher cette canule par un bouchon butyle adapté protégeant le produit à administrer jusqu'à injection. Le principal avantage de cet arrangement est de proposer une version avec une aiguille de diamètre désiré déjà installée, sans aucun contact avec un quelconque plastique.

De même, en comparaison avec la version précédemment décrite, avec aiguille standard fixée par son cône plastique, cette solution peut réduire aussi les volumes morts ainsi que les force d'injections requises pour administrer un même produit. On peut aussi envisager de l'associer à un dispositif test de veine en ménageant une ouverture ou fuite d'air entre la canule et le produit à injecter d'un diamètre trop petit pour qu'un produit visqueux puisse s'y écouler, mais assez grand pour que l'air y passe et fasse remonter le sang.

Cette solution à aiguille fixe peut s'adapter autant au système à vis décrit précédemment selon l'invention qu'à un arrangement du type seringue standard avec une tige de piston classique.

On a représenté sur la figure 10 un dispositif d'injection dont le corps 3 reçoit une cartouche standard 5 dont le septum 6 est destiné à être percé par une aiguille 7 lors de la mise en place de la cartouche 5 dans le corps 3. Dans cet exemple, et ainsi que représenté sur la figure 10a, l'aiguille 7 est constituée de deux parties, à savoir une partie externe et antérieure 7a de type classique et d'un diamètre interne $d1$ fonction du produit à injecter et de l'opération à réaliser et une partie interne et postérieure 37 de diamètre interne $d2$ plus important. Les deux parties antérieure 7a et postérieure 37 de l'aiguille 7 sont réunies entre elles par exemple par collage ou par soudure. Ainsi que représenté sur les figures 10 et 10a, la partie antérieure 7a de l'aiguille traverse la paroi distale du corps 3 de façon telle que sa partie postérieure 37 de plus grand diamètre vienne en contact avec la face interne de ladite paroi distale 3. L'extrémité libre de la partie postérieure 37 se termine par un biseau de façon à améliorer ses capacités de perforation du septum 6 lorsque l'on met celle-ci en place dans le corps 3.

On fera en sorte que la longueur m de la partie postérieure 37 de plus fort diamètre soit aussi réduite que possible, c'est-à-dire légèrement supérieure à l'épaisseur du septum de la cartouche que le corps 3 est destiné à recevoir, de façon à lui permettre de juste traverser ce dernier lorsque l'on met en place la cartouche.

On comprend ainsi que la partie postérieure 37 de plus grand diamètre assure plusieurs fonctions.

De première part elle assure un meilleur maintien de l'aiguille 7 par rapport à la partie distale du corps 3, dans la mesure où cette partie interne 37 forme une sorte d'épaulement.

De seconde part le diamètre $d2$ de son conduit interne étant supérieur au diamètre $d1$ du conduit de la partie antérieure 7a, la perte de charge subie par le produit injecté au cours de l'injection sera plus faible qu'elle ne l'est suivant l'état antérieur de la technique lorsque la partie postérieure 37 a le même diamètre que la partie externe.

Enfin, en donnant à cette partie postérieure 37 une longueur telle qu'elle permette juste de traverser le septum 6 on diminue encore la perte de charge subie par le produit au cours de l'injection et on diminue de ce fait la force d'injection nécessaire. Comme exposé précédemment cela permet soit de diminuer le diamètre interne de l'aiguille soit d'augmenter la longueur de celle-ci, soit d'injecter des produits moins fluides à travers un diamètre d'aiguille inférieur à ceux utilisés actuellement.

Bien que le dispositif d'injection suivant l'invention ait été décrit précédemment en regard d'exemples dans lesquels les réservoirs contenant le produit à injecter étaient constitués de cartouches, il peut bien entendu être également mis en œuvre avec des réservoirs constitués de seringues de type standard ou non.

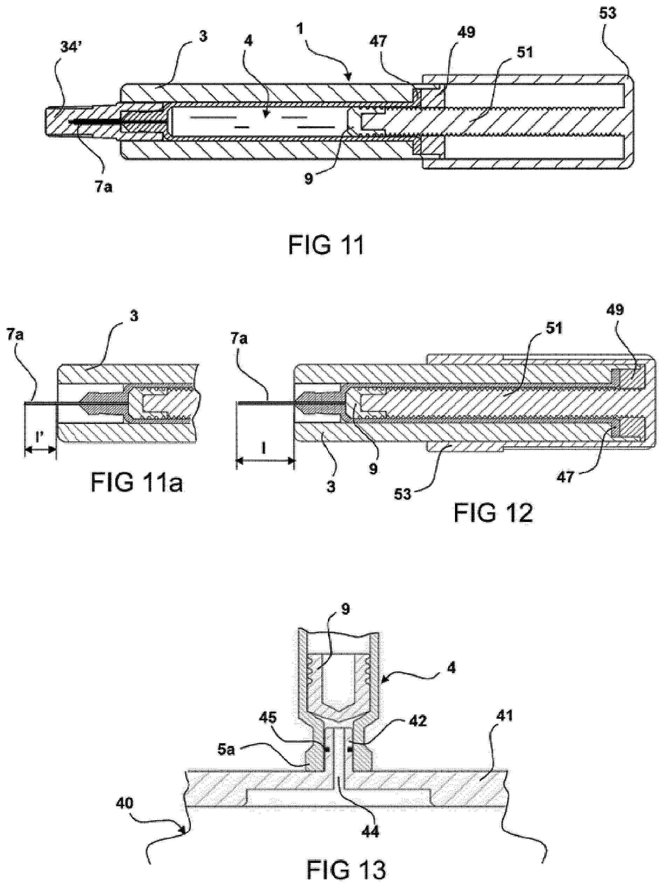

On a ainsi représenté sur les figures 11, 11a et 12 un tel dispositif d'injection 1 comportant un corps tubulaire 3 dans lequel est introduite une seringue standard 4 se terminant par une aiguille fixe dont la canule 7a est protégée par un capuchon étanche 34', notamment constitué de butyle. L'extrémité proximale 47 de cette seringue est maintenue dans le boîtier 3 par un écrou 49 qui est lui-même fixé sur l'extrémité proximale du corps 3. L'écrou 49 est traversé par un filetage dans lequel est vissée une tige filetée 51 dont l'extrémité distale est solidarisée du piston 9 de la seringue 4.

L'autre extrémité de la tige filetée 51 fait partie intégrante du fond d'un capuchon 53 en forme de cloche dont les dimensions sont telles, qu'en position retirée du piston (figure 11), il recouvre légèrement le corps 3. Dans ces conditions l'injection s'effectue par la simple rotation du capuchon 53 par rapport au corps 3.

On donnera à ce dernier une longueur telle que son extrémité distale arrive à l'extrémité implantable de la canule 7a de l'aiguille et découvre une longueur donnée l de celle-ci, ainsi que représenté sur les figures 11 et 12.

Par ailleurs, suivant l'invention, on comprend qu'en jouant sur la longueur du corps 3 on peut, pour une seringue donnée équipée d'une aiguille dont la canule 7a a une longueur donnée, régler la longueur l' de la partie implantable de celle-ci, ainsi que représenté sur la figure 11a.

Le présent dispositif d'injection permet ainsi d'une part de contrôler la longueur implantable de la canule 7a et de seconde part d'assurer un positionnement stable sur la peau du patient pendant la phase d'injection, notamment en raison de la surface d'appui du corps 3 sur celui-ci et de la préhension réalisée par l'utilisateur.

La présente demande décrit également un procédé et un dispositif de remplissage pour un système d'injection.

Dans un premier mode de mise en œuvre on a représenté sur la figure 13, des moyens permettant d'assurer le remplissage et le conditionnement d'un système d'injection 1 ou de son réservoir 5 à partir du col 5a de ce dernier, c'est-à-dire à l'inverse du sens de remplissage habituellement utilisé suivant l'état antérieur de la technique. Un tel procédé de remplissage est particulièrement intéressant lorsque le produit dont on souhaite remplir le réservoir 5 est d'une viscosité importante.

Ce dispositif de remplissage comporte un récipient de stockage 40 qui est pourvu d'une tête de remplissage 41 qui comporte une buse de connexion 42 d'un diamètre lui permettant d'être introduite dans le col 5a et qui est percée d'un conduit d'alimentation 44. L'étanchéité entre la buse 42 et le col 5a du dispositif d'injection peut être assurée par un joint, par exemple de type torique 45, prenant place dans une rainure circulaire de ladite buse 42.

Avant remplissage, ainsi que représenté sur la figure 13, le piston 9 du dispositif d'injection 1 se trouve en butée contre l'entrée du col 5a. Lorsque, lors de la phase de remplissage, l'on injecte le produit de traitement par le conduit d'alimentation 44, il repousse alors le piston 9, si bien que l'on réalise le remplissage de la dose de produit souhaité en évitant l'inconvénient de créer l'emprisonnement d'un volume d'air entre le piston et le produit de traitement.

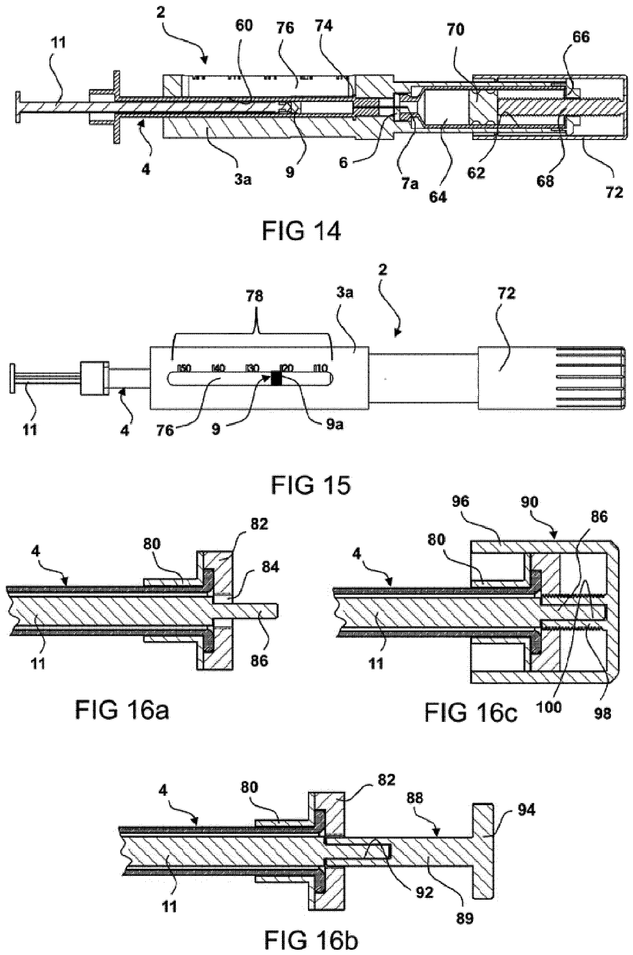

Dans un second mode de mise en œuvre on a représenté sur les figures 14 et 15 un dispositif de remplissage 2 d'une seringue 4 de type standard qui permet de remplir celle-ci à partir de son extrémité distale, à savoir au travers de la canule 7a de son aiguille.

Ce dispositif de remplissage est constitué d'un boîtier récepteur 3a de forme cylindrique qui est percé à chacune de ses extrémités d'un logement cylindrique longitudinal, à savoir un logement 60 réalisé dans sa partie proximale qui est apte à recevoir la seringue 4, et un logement 62 réalisé dans sa partie distale qui est apte à recevoir un réservoir de remplissage 64 qui est fermé de façon classique par un septum 6 du même type que celui représenté sur la figure 10.

Ce réservoir de remplissage 64 est maintenu en position dans le logement 62 par un écrou 66 qui est vissé dans le boîtier 3a et qui est percé d'un filetage dans lequel est vissée une tige filetée 68 dont l'extrémité distale est solidarisée d'un piston 70.

L'autre extrémité de la tige filetée 68 fait partie intégrante du fond d'un capuchon 72 en forme de cloche dont les dimensions sont telles qu'en position retirée du piston 70 il recouvre légèrement le boîtier 3a.

La partie distale du logement 60 recevant la seringue 4 se termine par un bossage 74 qui forme une butée contre laquelle la seringue vient en contact lors de son introduction dans ledit logement 60. Ce bossage 74 est disposé de façon telle que, lorsque la seringue 4 est en contact avec lui sa canule 7a a perforé le septum 6 du réservoir de remplissage 64 et a pénétré dans ce dernier.

Dans ces conditions, le remplissage de la seringue avec une dose déterminée de produit de traitement s'effectue de la façon suivante. On repousse tout d'abord la tige de piston 11 de la seringue 4 de façon que son piston 9 soit en butée contre le fond de celle-ci, puis on met en rotation le capuchon 72, ce qui a pour effet de repousser le piston 70 et d'expulser une quantité donnée de produit de traitement dans la seringue 4 au travers de sa canule 7a en repoussant le piston 9 de celle-ci.

On comprend que ce mode de remplissage présente un premier avantage d'éviter la création d'une poche d'air entre le piston 9 de la seringue et le produit de traitement.

Par ailleurs, on peut contrôler la quantité de produit de traitement injecté dans la seringue 4 à l'aide de moyens permettant de contrôler le déplacement longitudinal du capuchon 72 par rapport au boîtier 3a, ces moyens pouvant être du type de ceux utilisés dans le dispositif en regard de la figure 9.

Ainsi que représenté sur la figure 15 on pourra préférentiellement réaliser au travers du boîtier 3a une fenêtre longitudinale 76 permettant de voir au travers de celle-ci la seringue 4 et notamment la position de son piston 9. On réalisera également sur le boîtier 3a, et en bordure de la fenêtre 76, des graduations 78 qui seront espacées de façon telle qu'elles correspondent à des doses de produit à injecter. L'utilisateur de la seringue vissera ainsi le capuchon 72 jusqu'à ce que la partie distale 9a du piston 9 de la seringue se trouve en face de la graduation 78 correspondant à la quantité de produit de traitement qu'il souhaite injecter, à savoir par exemple 20 unités sur la figure 15.

Une fois la dose souhaitée chargée dans la seringue l'utilisateur retirera celle-ci du dispositif de remplissage 2 et pourra pratiquer l'injection. Il pourra recommencer facilement avec une autre seringue en introduisant cette dernière dans le logement 60 du dispositif de remplissage 2 et en procédant ainsi qu'indiqué précédemment.

Ce procédé de remplissage d'une seringue notamment de type standard et le dispositif de remplissage utilisé pour le mettre en œuvre sont tout particulièrement intéressants lorsque l'on doit procéder à des injections répétées ou de quantités variables quotidiennes, notamment de produits de traitement tels que l'insuline ou les hormones de croissance, en raison de la facilité de la mise en œuvre qui les rend utilisables par le patient luimême.

Par ailleurs on a pu vérifier lors d'essais que cet arrangement donne une précision comparable à celle fournie par les stylos-injecteurs classiques et facilite de plus l'injection de grands volumes de produit de traitement sans être soumis aux limites de doses et contraintes de temps d'injection desdits stylo-injecteurs. Cela permet de pratiquer une injection manuelle à partir d'un dispositif d'injection rempli par des moyens commandés par rotation qui peut remplir toutes les fonctions des stylo-injecteurs classiques en utilisant de simples seringues standards et qui peut être encore plus petit, simple, léger robuste et donc moins cher.

On sait que pour certains produits injectables il peut être nécessaire de procéder avant l'injection à un mélange de deux produits de base, notamment dans le cas d'une préparation extemporanée, comme par exemple une reconstitution pour certaines formulations composées. Il peut aussi être nécessaire de transférer tout ou partie d'un produit de traitement depuis un conditionnement primaire dans un réservoir qui sera ensuite utilisé pour réaliser l'injection.

La méthode la plus habituelle consiste à utiliser deux seringues contenant chacune l'un des deux produits à mélanger que l'on réunit ensemble au moyen d'un connecteur. On effectue ensuite un va-et-vient des produits pour les faire passer d'une seringue à l'autre, et obtenir ainsi un mélange homogène. Par la suite, tout le produit ainsi mélangé est transféré dans l'une des deux seringues qui servira de seringue d'injection.

On comprend que si les multiples transferts des produits de base d'une seringue à l'autre peuvent être effectués facilement et rapidement lorsque les tiges de piston de ces seringues sont actionnées par pression, il n'en est pas de même lorsque ces tiges de piston sont actionnées par rotation ainsi qu'il en est par exemple dans les dispositifs décrits à titre d'exemples illustrés par les figures 8, 9, 11 et 14.

La présente invention telle que représentée sur les figures 16a, 16b et 16c permet de remédier à cet inconvénient et permet d'assurer facilement le mélange avant injection de deux produits de base tout en permettant une injection par une action rotative sur sa tige de piston.

Ce dispositif d'injection est constitué en l'espèce d'une seringue 4 dont l'extrémité proximale est maintenue entre une bride circulaire 80 et une plaque de maintien 82 solidarisée de celle-ci par tout moyen, non représenté sur le dessin, et qui est percée d'un trou fileté 84 dont le diamètre est légèrement supérieur à celui de la tige de piston 11 de cette seringue. L'extrémité proximale de cette tige de piston 11 se termine par un téton cylindrique 86 d'un diamètre inférieur à celui de la tige de piston 11. Le dispositif d'injection comprend ainsi, outre la seringue 4, deux têtes de commande, à savoir une tête de commande par pression 88 et une tête de commande par rotation 90.

La tête de commande par pression 88 est constituée d'une tige cylindrique 89 d'un diamètre inférieur au trou fileté 84 et voisin de celui de la tige de piston 11, cette tige cylindrique étant creusée en son extrémité distale d'un logement axial 92 apte à lui permettre de recevoir le téton 86 et dont l'extrémité proximale est formée d'un méplat 94, ainsi que représenté sur la figure 16b. On comprend, qu'ainsi constitué, le dispositif d'injection est apte à fonctionner comme une seringue commandée par pression, ce qui le rend apte à effectuer facilement et rapidement les transferts de seringue à seringue précédemment mentionnés.

La tête de commande par rotation 90 comporte, ainsi que dans les réalisations précédentes illustrées sur les figures précédemment citées, un capuchon 96 en forme de cloche dont le fond comporte une portion de vis axiale 98 apte à se visser dans le trou fileté 84 et qui est percé d'un logement axial 100 apte à recevoir le téton 86, ainsi que représenté sur la figure 16c. On comprend qu'ainsi, constitué, le dispositif d'injection est apte à fonctionner comme une seringue commandée par rotation, puisqu'une rotation dans le sens du vissage du capuchon 96 a pour effet de repousser la tige de piston 11 provoquant ainsi l'injection du produit de traitement contenu dans la seringue 4.

Le présent mode de réalisation est intéressant en ce que, par un simple échange de la tête de commande, l'utilisateur peut effectuer facilement et rapidement un va et vient de produits de seringue à seringue nécessaire à un mélange et une injection de produit de traitement au moyen d'un dispositif d'injection à commande par rotation.

On sait que lorsque les dispositifs d'injection classiques, tels que notamment les seringues ou les cartouches, simples ou à double chambre, sont utilisés pour réaliser des injections en intra-musculaire il est nécessaire de s'assurer que l'administration n'est pas réalisée dans une veine. C'est pourquoi, une fois l'aiguille implantée, le praticien réalise une vérification, dite « test de veine », en effectuant une aspiration avec le piston de la seringue. En cas d'un retour sanguin, qui se traduit par une arrivée de sang dans la seringue, il constate que l'aiguille se trouve dans une veine et que, en conséquence, il ne peut pas pratiquer l'injection.

Dans les dispositifs d'injection dans lesquels l'injection se fait non par pression mais par rotation l'aspiration effectuée par le retrait du piston s'avère plus délicate à réaliser, surtout dans les cas où les produits d'injection sont visqueux ou très visqueux.

Cependant la demanderesse a constaté, que dans le cas d'une cartouche en verre par exemple, lorsque la bague de sertissage est scellée contre le col du réservoir, le contact entre ces deux composants, de par leur composition métal sur verre, s'il empêche le passage de liquide ou semi-solides n'est pas totalement étanche à l'air. Dans ces conditions, si l'utilisateur implante l'aiguille dans un vaisseau sanguin, la pression sanguine fait remonter le sang dans l'aiguille, l'air s'échappant par la jonction réservoir-bague de sertissage. Le sang prend alors la place de l'air dans le réservoir, teintant sa partie distale visible au praticien. Comme dans le cas d'un dispositif d'injection standard de type seringue, la présence de sang dans le réservoir indique alors qu'il n'est pas possible de réaliser l'injection à cet endroit précis.

Dans ces conditions on comprend que les modes d'injection par rotation suivant l'invention permettent la réalisation du test de veine, et ce de façon automatique.

On a représenté sur la figure 17 un dispositif destiné au conditionnement de seringues ou cartouches désigné communément sous le nom de « nest » et qui sert habituellement pour réaliser le remplissage de produits sous forme liquide ou peu visqueux.

On utilise suivant l'invention ce dispositif pour assurer le conditionnement d'une série de seringues ou de cartouches destinées à contenir notamment un premier produit de base destiné à être ultérieurement mélangé avec un second produit de base afin de réaliser une préparation extemporanée d'un produit de traitement injectable, le premier produit de base pouvant notamment se présenter sous forme sèche ou lyophilisée.

Le dispositif de conditionnement comprend ainsi le « nest » qui, de façon connue comporte un bac 102 fermé par un couvercle 104 et qui contient des moyens aptes à maintenir vertical un ensemble de réservoirs, constitués de cartouches ou de seringues 4, que l'on souhaite remplir et conditionner. Le couvercle 104 est muni de moyens de centrage en position qui sont constitués de quatre plots mâles 106 fixés dans ses angles et sous sa face inférieure et qui sont aptes à être reçus dans quatre plots femelles 108 disposés sur le bac 102 en vis-à-vis des plots mâles. Des ressorts de compression hélicoïdaux 110 entourent les plots mâles et femelles et assurent, lorsqu'ils sont en position non comprimée (figure 17), le maintien en position ouverte du couvercle 104.

Suivant l'invention la surface inférieure du couvercle 104 est pourvue, au droit de chacune des seringues 4, d'un picot 112 perpendiculaire à la surface du couvercle qui est destiné à recevoir à friction un piston destiné à constituer ultérieurement le piston 9 de la seringue 4.

Le procédé de conditionnement du dispositif d'injection s'effectue ainsi que décrit ci-après. On procède tout d'abord au remplissage des seringues avec tout dispositif connu conventionnel, puis on procède aux opérations de séchage ou lyophilisation éventuelles, les ressorts 110 maintenant le couvercle 104 en position pendant cette opération. En fin de séchage ou de lyophilisation on exerce une pression P sur la surface externe du couvercle 104, à l'encontre de la force exercée par les ressorts 110, si bien que l'on introduit chaque piston 9 dans le réservoir de la seringue 4 avec lequel il est en vis-à-vis. Cette opération peut être réalisée à l'intérieur d'une chambre ou d'un lyophilisateur gardé sous un vide calculé d'air ou de gaz inerte. On peut ensuite casser le vide pour revenir à la pression ambiante, si bien que cette dernière exerce une force sur chaque piston 9 qui a pour effet de le maintenir dans chaque réservoir lorsque l'on cesse la pression P appliquée au couvercle 104 et que chaque ressort 110 agit pour remonter celui-ci. Le produit se trouve alors conditionné dans les seringues 4 et pourra être mélangé ensuite avec une fraction liquide pour être ensuite injecté en utilisant préférentiellement un dispositif selon l'invention tel que décrit précédemment sur les Figues 16a, 16b et 16c.

La présente demande décrit également un dispositif d'injection de sécurité passif destiné à permettre une stabilité accrue de son positionnement lors de l'injection et une protection de son aiguille d'injection avant pendant et après l'injection.

Un tel dispositif de sécurité est dit passif en raison de ce que d'une part son fonctionnement est entièrement indépendant de la façon dont l'utilisateur opère pour réaliser l'injection et d'autre part celui-ci ne peut pas réaliser d'opérations ou d'erreurs qui empêcheraient son déclenchement comme par exemple de ne pas aller au bout d'une injection avant de retirer l'aiguille.

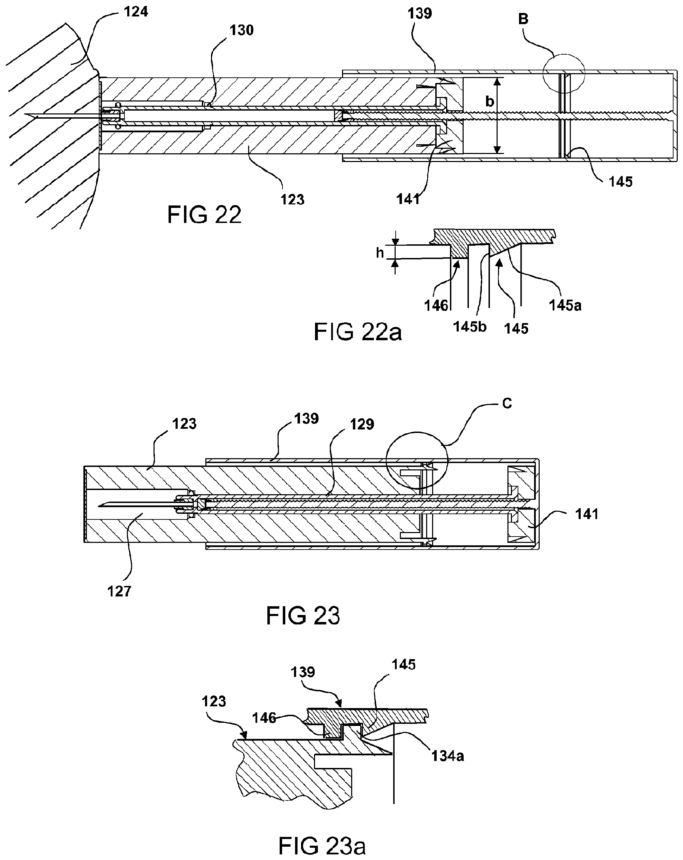

On a représenté sur les figures 20 à 23a un mode de mise en œuvre d'un tel dispositif d'injection.

Ce dispositif d'injection 121 comprend un corps tubulaire 123 qui est creusé d'un conduit cylindrique axial 125 qui débouche dans une chambre distale cylindrique 127 de plus grand diamètre. Le conduit 125 reçoit un réservoir, constitué dans ce mode de mise en œuvre, par une seringue 129 contenant le produit à injecter qui se termine par une aiguille d'injection 131. L'extrémité distale de la seringue 129 est pourvue d'un joint torique d'étanchéité 132 qui est encastré dans une cuvette 130 du corps 123, et la longueur a de la chambre 127 est telle, qu'en position de repos, ou pré injection, ainsi que représenté sur la figure 1, l'aiguille d'injection 131 se trouve logée à l'intérieur de la chambre 127.

L'extrémité proximale 123b du corps 123 se termine par deux languettes d'accrochage 134 diamétralement opposées qui se terminent elles-mêmes par un bossage externe 134a de hauteur H suivi d'un biseau 134b, ces languettes étant rendues déformables par la présence d'un espace interne 135.

Un piston 136 est monté coulissant à l'intérieur de la seringue 129 sous l'action d'une tige de piston 137 filetée. L'extrémité proximale de cette tige de piston est solidaire du fond d'un organe d'actionnement constitué d'un capuchon cylindrique 139 dont le diamètre interne D est légèrement supérieur à celui d du corps 123 augmenté de la hauteur des deux butées 134a, de façon à permettre le coulissement du capuchon 139 sur le corps. On a ainsi D = d + 2xH. En position de repos, (cf figure 1) l'extrémité distale de ce capuchon 139 est légèrement engagée sur le corps 123.

La face interne du capuchon 139 comporte une première butée distale circulaire 146 de hauteur h, de coupe rectangulaire, visant à maintenir le corps 123 bloqué dans le capuchon 139 lors du retrait de l'aiguille du corps 124 une fois l'administration du produit terminée. Enfin, la face interne du capuchon comprend une seconde butée proximale circulaire 145 de hauteur h dont la face proximale 145a est préférentiellement inclinée et la face distale 145b est radiale, et écartée de la première butée 145 d'une distance égale à la largeur du bossage 134a, et qui est destinée, ainsi qu'expliqué ci-après, à interdire une seconde sortie de l'aiguille 131 une fois le processus d'injection terminé.

Un écrou cylindrique 141, d'épaisseur e, formant butée est vissé sur la tige de piston 137 et comporte une cuvette 140 dans laquelle l'extrémité proximale de la seringue 129 est fixée. La périphérie de la face distale de l'écrou 141 est creusée d'une rainure circulaire 143 de forme complémentaire de celle du biseau 134b des languettes déformables 134. Le diamètre b de cet écrou 141 est légèrement inférieur au diamètre interne D du capuchon 139 auquel on a retranché les hauteurs H des deux bossages 134a. On a ainsi b = D - 2xH.

La distance c qui existe, lorsque le dispositif d'injection est au repos, ainsi que représenté sur la figure 1, entre l'extrémité proximale 123b du corps 123 et la face distale de l'écrou 141 permet de contrôler la distance dont l'aiguille d'injection émergera de la face distale 123a du corps 123 lors de l'injection, ainsi qu'expliqué ci-après.

Cette face distale 123a du corps cylindrique 123 peut être formée par la surface annulaire du corps 123 ou, comme dans le mode de mise en œuvre représenté sur la figure 2, comporter une partie d'extension 123c permettant d'augmenter l'importance de cette surface.

Cette dernière est revêtue d'un adhésif apte à permettre une adhérence avec la peau 124 du patient à traiter. On utilisera ainsi par exemple des surfaces collantes à base de silicone, d'acrylate, de polyacrylates, ou tout autre copolymères connus entrant dans la composition de surface adhésives pour dispositifs médicaux, comme par exemples les pansements, les poches de stomie, les électrodes et plaques électro-chirurgicales, les patchs ou encore les prothèses capillaires. Cette face adhésive sera protégée par une pellicule ou un film 147.

Dans ces conditions le fonctionnement du dispositif d'injection suivant l'invention s'établit ainsi que décrit ci-après.

Après avoir retiré le film protecteur 147, l'utilisateur met en contact l'extrémité distale adhésive du dispositif d'injection avec la zone du corps du patient, là où il souhaite effectuer l'injection. Dès lors cette extrémité du dispositif d'injection, du fait de son adhérence avec la zone d'injection, se trouve stabilisée.

Ensuite, l'utilisateur exerce une poussée linéaire sur le capuchon 139 dans le sens de la flèche F jusqu'à ce que l'écrou 141 vienne en butée avec la face proximale 123b du corps 123, ce qui a pour effet de première part de faire sortir l'aiguille d'injection 131 du dispositif d'injection et de l'introduire dans le corps 124 du patient, ainsi que représenté sur la figure 3. D'autre part, en fin de ce mouvement de translation, les biseaux 134b des languettes 134 pénètrent dans la rainure 143 de l'écrou, ce qui a pour effet de déformer vers l'intérieur les languettes 134 et d'assurer une liaison en rotation de l'écrou 141 avec le corps 123. De plus, en raison de la déformation vers l'intérieur des languettes, celles-ci se trouvent dégagées et en mesure de ne pas bloquer le déplacement du capuchon lorsque, lors de l'injection, elles arriveront au niveau des butées circulaires 145 et 146.

Dès lors le dispositif d'injection est prêt pour procéder à l'injection du produit de traitement.

Pour ce faire l'utilisateur met en rotation le capuchon 139, ce qu'il peut d'ailleurs effectuer d'une seule main en raison de la liaison existant entre la partie distale 123a du corps 123 avec le corps 124 du patient qui est assurée par le produit adhésif. Lors de cette rotation du capuchon 139, la tige de clapet 137 se visse dans l'écrou 141, et celui-ci étant immobilisé en rotation par rapport au corps 123, le piston 136 se déplace dans la seringue 129 et administre le produit de traitement dans le corps du patient jusqu'à ce que le piston atteigne le col de la seringue 129, ou que l'utilisateur arrête son injection.

Une fois l'injection effectuée, l'utilisateur effectue une traction sur le capuchon 139 en sens inverse de la flèche F et, la face distale 123a étant en adhérence avec la peau du patient, cette traction a pour effet de ramener en arrière la seringue 129 ainsi que l'écrou 141, si bien que les languettes 134 se trouvent alors libérées et reprennent leur position initiale. Le mouvement de retrait s'effectue donc jusqu'à ce que la face proximale du bossage 134a des languettes 134 vienne en contact avec la butée 145, et tombe dans l'espace libre compris entre les deux butées 145 et 146 ainsi que représenté sur les figures 4 et 4a.

Dès lors tout mouvement relatif du capuchon 139 par rapport au corps 123 dans la direction de la flèche F ou inverse n'est plus possible et le dispositif d'injection se trouve ainsi verrouillé et sécurisé contre toute utilisation ultérieure.

En poursuivant le mouvement de retrait l'utilisateur assure le décollement de la face distale du dispositif d'injection de la surface de la peau du patient grâce au blocage du corps 123 par rapport au capuchon 139 contre la seconde butée distale 146.

On notera que les moyens de sécurité qui équipent le présent dispositif d'injection présentent l'avantage d'être beaucoup plus simples et donc plus faciles et moins chers à réaliser que les systèmes actuels basés par exemple sur le déclenchement de mécanismes à ressort.

Bien entendu et bien que le présent exemple soit décrit en regard d'une seringue de petit volume, notamment de type à insuline, il peut s'adapter à tout autre dispositif faisant appel à des seringues ou cartouches, standard ou non, que ce soit selon un mode d'injection à vis ainsi que décrit dans le présent exemple ou selon des modes d'injection en translation traditionnels avec des seringues classiques faisant appel à des tiges de piston lisses et des appui-doigts.

Dans une telle version avec tige de piston lisse actionnée en translation, le capuchon 139 est remplacé par deux bras également solidaires de la tige de piston. Ces deux bras peuvent circuler dans deux fenêtres à l'intérieur de l'appui-doigts. Comme pour la version à vis, l'implantation de l'aiguille se fait en exerçant une poussée de ces deux bras dans le sens de la flèche F.

De plus les moyens de sécurité mis en œuvre dans le présent dispositif d'injection présentent également l'avantage de pouvoir remplacer le capuchon qui recouvre habituellement l'aiguille d'injection. Cela supprime une opération de retrait du capuchon qui est dangereuse en raison de ce qu'elle expose l'utilisateur au biseau coupant, et que de plus elle peut endommager l'aiguille d'injection et qu'enfin elle comporte des risques de contaminations extérieures de cette aiguille avant son injection.

Dans une variante de mise en œuvre de l'invention le corps 123 pourra comporter une fenêtre qui s'étendra le long de la seringue et qui permettra de vérifier son contenu avant utilisation.

Par ailleurs, et suivant l'invention une étiquette pourra relier le corps 123 au capuchon 139 de façon à assurer le maintien relatif de ces deux parties avant l'injection. Une telle étiquette permettra également de vérifier avant l'utilisation l'intégrité du scellage du dispositif d'injection. Cette étiquette pourra comporter une ligne de prédécoupe favorisant sa rupture lors de l'injection.

**Revendications**

**1.** Dispositif d'injection du type comportant un corps (3) cylindrique renfermant un réservoir (5) contenant un produit à injecter, qui est ouvert à une extrémité sur une aiguille d'injection (7) pourvue d'une canule (7a) et qui est fermé à son autre extrémité par un piston (9) monté mobile en translation dans le réservoir sous la commande d'une tige de piston (11,51) pourvue d'un organe de commande (13,13',53), ce dispositif comportant des moyens de contrôle de la quantité de produit à délivrer, lesdits moyens de contrôle étant disposés sur le corps (3) en une zone se situant hors de la zone de préhension à pleine main du corps qui est située dans la zone distale (16) du corps(3), **caractérisé en ce que** lesdits moyens de contrôle comprennent une tige de piston (11) comportant une partie filetée (11a) sur laquelle est vissée une molette de réglage (15), et **en ce que** le corps (3) comporte deux butées extrêmes, à savoir une butée distale (17a) et une butée proximale (17b) entre lesquelles la molette de réglage (15) peut être positionnée, ce dispositif permettant à l'utilisateur de sélectionner à l'avance la quantité de produit qui sera injectée à un patient sans avoir, au cours de l'injection, à contrôler la quantité de produit délivré.

**2.** Dispositif d'injection suivant la revendication 1, **caractérisé en ce que** la molette de réglage (15) comporte, sur une zone périphérique, des graduations (18), qui sont visibles par l'extrémité proximale du corps (3), et ce dernier comporte au moins un index (20) dont la coopération avec les graduations (18) permet de déterminer un angle de rotation de ladite molette de réglage (15) et ainsi son déplacement longitudinal par rapport à la partie filetée (11a).

**3.** Dispositif d'injection suivant la revendication 2, **caractérisé en ce que** la zone de la molette de réglage (15) comportant les graduations (18) dépasse latéralement du corps (3) en au moins une zone.

**4.** Dispositif d'injection suivant la revendication 2, **caractérisé en ce que** la partie proximale du corps (3) recouvre au moins en partie la molette de réglage (15) et comporte au moins une zone transparente au travers de laquelle on peut apercevoir au moins une des graduations (18).

**5.** Dispositif d'injection suivant la revendication 1, **caractérisé en ce que** la partie proximale du corps (3) forme un boîtier cylindrique (32), notamment ouvert sur l'un de ses côtés, qui est traversé par une partie filetée (11a) de la tige de piston (11), la molette de réglage (15') étant apte à se déplacer par vissage ou dévissage sur la partie filetée (11a) pour aller de la butée proximale (17b) à la butée distale (17a), ces butées étant respectivement constituées par les deux parois extrêmes du boîtier (32) et **en ce que** le chant (20') de la molette de réglage (15') constitue un index et des graduations (18) sont portées sur le corps (3) du boîtier (32).

6. Dispositif d'injection suivant l'une quelconque des revendications précédentes, **caractérisé en ce que** le réservoir (5) est constitué d'une cartouche ou d'une seringue standard d'injection.

7. Dispositif d'injection suivant l'une des revendications précédentes, **caractérisé en ce que** la tige de piston (11) comporte un élément de friction avec la paroi interne du réservoir (5), tel que notamment un joint torique (22).

8. Dispositif d'injection suivant la revendication 1 comprenant un réservoir (4), notamment une cartouche ou une seringue, dont le piston (9) est mu par la tige de piston (11) qui est de section droite circulaire sous l'action d'une tête de commande (88,90) et, à son extrémité distale, une aiguille d'injection, l'extrémité proximale de la tige de piston (11) comportant des moyens aptes à coopérer successivement, de façon amovible, avec d'une part une tête de commande de piston à pression (88) et, d'autre part une tête de commande de piston à rotation (90) et en ce que le dispositif comporte un écrou (82) apte à se fixer sur la partie proximale du réservoir (4) dont le diamètre du trou fileté est supérieur à celui de la tige de piston (11) et est inférieur au diamètre interne du réservoir (4) **caractérisé en ce que** la tête de commande de piston à pression (88) est constituée d'un élément cylindrique (89) dont le diamètre est inférieur à celui du trou fileté de l'écrou (82), de façon qu'il puisse être reçu dans le réservoir (4), cet élément cylindrique (89) étant apte à se solidariser de l'extrémité proximale (86) de la tige de piston (11).

9. Dispositif d'injection suivant la revendication 8, **caractérisé en ce que** la tête de commande de piston à rotation (90) comprend une vis (98) apte à se visser dans le trou fileté de l'écrou (82), de façon à venir en appui sur la partie proximale de la tige de piston (11) et être en mesure de la repousser vers l'extrémité distale du réservoir (4) lorsque l'on visse la vis (98) et **en ce que** la vis (98) est solidarisée du fond d'un capuchon (96).

10. Dispositif d'injection suivant l'une quelconque des revendications précédentes, **caractérisé en ce qu'**il comporte une aiguille d'injection (7) pourvue d'une canule (7a) creusée d'un canal, cette canule (7a) étant solidarisée d'une embase (36), comportant un plateau circulaire (36a) axialement centré avec un bossage cylindrique adjacent (36b) de même axe (xx'), qui est creusé d'une cavité (36c) en communication avec le canal de l'aiguille, cette cavité (36c) formant un conduit convergent en allant de sa partie proximale vers sa partie distale.

11. Dispositif d'injection suivant l'une quelconque des revendications 1 à 10, **caractérisé en ce qu'**il comporte une aiguille d'injection (7), pourvue d'une canule (7a), creusée d'un canal, cette canule (7a) étant solidaire d'une embase cylindrique (37) formée d'un élément tubulaire comportant un canal (7") disposé dans le prolongement de celui de la canule (7a), et dont le diamètre interne (d2) est supérieur à celui (d1) de cette dernière et **en ce que** le diamètre externe de l'embase (37) est supérieur à celui de la canule (7a) .

**Patentansprüche**

1. Injektionsvorrichtung des Typs, der einen zylindrischen Körper (3) umfasst, der einen Behälter (5) umschließt, der ein zu injizierendes Produkt enthält, der an einem Ende zu einer Injektionsnadel (7) hin offen ist, die mit einer Kanüle (7a) versehen ist, und der an seinem anderen Ende durch einen Kolben (9) verschlossen ist, der translatorisch beweglich in dem Behälter montiert ist, unter der Steuerung einer Kolbenstange (11, 51), die mit einem Steuerorgan (13, 13', 53) versehen ist, wobei diese Vorrichtung Mittel zur Kontrolle der Menge des abzugebenden Produkts umfasst, wobei die Kontrollmittel auf dem Körper (3) in einem Bereich angeordnet sind, der sich außerhalb des Bereichs befindet, an dem der Körper mit der ganzen Hand ergriffen werden kann, der sich im distalen Bereich (16) des Körpers (3) befindet, **dadurch gekennzeichnet, dass** die Kontrollmittel eine Kolbenstange (11) aufweisen, die einen Gewindeteil (11a) umfasst, auf den ein Einstellrad (15) aufgeschraubt ist, und dadurch, dass der Körper (3) zwei Endanschläge umfasst, nämlich einen distalen Anschlag (17a) und einen proximalen Anschlag (17b), zwischen denen das Einstellrad (15) positioniert werden kann, wobei diese Vorrichtung es dem Benutzer ermöglicht, die Produktmenge, die einem Patienten injiziert wird, im Voraus auszuwählen, ohne während der Injektion die Menge des abgegebenen Produkts kontrollieren zu müssen.

2. Injektionsvorrichtung nach Anspruch 1, **dadurch gekennzeichnet, dass** das Einstellrad (15) in einem Umfangsbereich Skalen (18) umfasst, die durch das proximale Ende des Körpers (3) sichtbar sind, und dieser mindestens einen Zeiger (20) umfasst, dessen Zusammenwirken mit den Skalen (18) es ermöglicht, einen Drehwinkel des Einstellrades (15) und somit seine Längsverschiebung in Bezug auf den Gewindeteil (11a) zu bestimmen.

3. Injektionsvorrichtung nach Anspruch 2, **dadurch gekennzeichnet, dass** der Bereich des Einstellrads (15), der die Skalen (18) umfasst, an mindestens einem Bereich seitlich über den Körper (3) hinausragt.

**4.** Injektionsvorrichtung nach Anspruch 2, **dadurch gekennzeichnet, dass** der proximale Teil des Körpers (3) mindestens teilweise das Einstellrad (15) abdeckt und mindestens einen transparenten Bereich umfasst, durch den man mindestens eine der Skalen (18) sehen kann.

**5.** Injektionsvorrichtung nach Anspruch 1, **dadurch gekennzeichnet, dass** der proximale Teil des Körpers (3) ein zylindrisches Gehäuse (32) bildet, das insbesondere an einer seiner Seiten offen ist und das von einem Gewindeteil (11a) der Kolbenstange (11) durchdrungen wird, wobei das Einstellrad (15') durch Auf- oder Abschrauben auf dem Gewindeteil (11a) verschiebbar ist, um vom proximalen Anschlag (17b) zum distalen Anschlag (17a) zu gelangen, wobei diese Anschläge jeweils von den beiden Endwänden des Gehäuses (32) gebildet werden, und dadurch, dass die Kante (20') des Einstellrades (15') einen Zeiger bildet und Skalen (18) auf dem Körper (3) des Gehäuses (32) angebracht sind.

**6.** Injektionsvorrichtung nach einem der vorhergehenden Ansprüche, **dadurch gekennzeichnet, dass** der Behälter (5) aus einer Kartusche oder einer Standard-Injektionsspritze besteht.

**7.** Injektionsvorrichtung nach einem der vorhergehenden Ansprüche, **dadurch gekennzeichnet, dass** die Kolbenstange (11) ein Element zur Reibung mit der Innenwand des Behälters (5) umfasst, wie insbesondere einen O-Ring (22).

**8.** Injektionsvorrichtung nach Anspruch 1, die einen Behälter (4), insbesondere eine Kartusche oder eine Spritze, dessen Kolben (9) durch die Kolbenstange (11), die einen kreisförmigen Querschnitt aufweist, unter der Wirkung eines Steuerkopfes (88, 90) bewegt wird, und an ihrem distalen Ende, eine Injektionsnadel aufweist, wobei das proximale Ende der Kolbenstange (11) Mittel umfasst, die geeignet sind, nacheinander lösbar mit einerseits einem Steuerkopf für den Druckkolben (88) und andererseits einem Steuerkopf des Drehkolbens (90) zusammenzuwirken, und dadurch, dass die Vorrichtung eine Mutter (82) umfasst, die geeignet ist, auf dem proximalen Teil des Behälters (4) befestigt zu werden, deren Durchmesser der Gewindebohrung größer als der der Kolbenstange (11) und kleiner als der Innendurchmesser des Behälters (4) ist, **dadurch gekennzeichnet, dass** der Steuerkopf des Druckkolbens (88) aus einem zylindrischen Element (89) besteht, dessen Durchmesser kleiner als der der Gewindebohrung der Mutter (82) ist, so dass er in dem Behälter (4) aufgenommen werden kann, wobei dieses zylindrische Element (89) geeignet ist, mit dem proximalen Ende (86) der Kolbenstange (11) fest verbunden zu werden.

**9.** Injektionsvorrichtung nach Anspruch 8, **dadurch gekennzeichnet, dass** der Steuerkopf des Drehkolbens (90) eine Schraube (98) aufweist, die geeignet ist, in die Gewindebohrung der Mutter (82) eingeschraubt zu werden, so dass sie auf dem proximalen Teil der Kolbenstange (11) zur Anlage kommt und in der Lage ist, diese zum distalen Ende des Behälters (4) hin zu drücken, wenn man die Schraube (98) eindreht, und dadurch, dass die Schraube (98) mit dem Boden einer Kappe (96) fest verbunden ist.

**10.** Injektionsvorrichtung nach einem der vorhergehenden Ansprüche, **dadurch gekennzeichnet, dass** sie eine Injektionsnadel (7) umfasst, die mit einer Kanüle (7a) versehen ist, die von einem Kanal durchzogen ist, wobei diese Kanüle (7a) fest mit einer Basis (36) verbunden ist, die eine kreisförmige Platte (36a) umfasst, die axial mit einem benachbarten zylindrischen Vorsprung (36b) mit derselben Achse (xx') zentriert ist, der von einem Hohlraum (36c) durchzogen ist, der mit dem Kanal der Nadel in Verbindung steht, wobei dieser Hohlraum (36c) einen konvergierenden Kanal bildet, der von seinem proximalen Teil zu seinem distalen Teil verläuft.

**11.** Injektionsvorrichtung nach einem der Ansprüche 1 bis 10, **dadurch gekennzeichnet, dass** sie eine Injektionsnadel (7) umfasst, die mit einer Kanüle (7a) versehen ist, die von einem Kanal durchzogen ist, wobei diese Kanüle (7a) fest mit einer zylindrischen Basis (37) verbunden ist, die aus einem rohrförmigen Element gebildet ist, das einen Kanal (7") umfasst, der in der Verlängerung desjenigen der Kanüle (7a) angeordnet ist und dessen Innendurchmesser (d2) größer ist als derjenige (d1) dieser letzteren, und dadurch, dass der Außendurchmesser der Basis (37) größer ist als derjenige der Kanüle (7a).

## Claims

**1.** An injection device of the type comprising a cylindrical body (3) enclosing a reservoir (5) containing a product to be injected, said reservoir opening at one end on an injection needle (7) provided with a cannula (7a) and being closed at its other end by a plunger (9) mounted so as to be movable in translation in the reservoir under the control of a plunger rod (11, 51) provided with a control member (13, 13', 53), the device comprising means for controlling the quantity of product to be delivered, said control means being arranged on the body (3) in an area located outside the area intended to be fully gripped by the hand which is located in the distal region (16) of the body (3) **characterised in that** said control means comprise a plunger rod (11) comprising a threaded portion (11a) on which an adjusting wheel (15) is screwed, and the body (3) comprises two end stops, namely a distal stop (17a) and a proximal stop (17b), between which the adjusting wheel (15) can be positioned, said device allowing the user to select in advance the quantity of product that will be injected into a patient without having, during the injection, to control the quantity of product delivered.

2. An injection device according to claim 1, **characterised in that** the adjusting wheel (15) comprises, in a peripheral area, graduations (18) which are visible from the proximal end of the body (3), and the latter comprises at least one index (20) whose cooperation with the graduations (18) makes it possible to determine an angle of rotation of said adjusting wheel (15) and thus its longitudinal displacement with respect to the threaded portion (11a).

3. An injection device according to claim 2, **characterised in that** the area of the adjusting wheel (15) comprising the graduations (18) projects laterally from the body (3) in at least one area.

4. An injection device according to claim 2, **characterised in that** the proximal part of the body (3) at least partially covers the adjusting wheel (15) and comprises at least one transparent area through which at least one of the graduations (18) can be seen.

5. An injection device according to claim 1, **characterised in that** the proximal part of the body (3) forms a cylindrical housing (32), especially open on one of its sides, which is penetrated by a threaded portion (11a) of the plunger rod (11), the adjusting wheel (15') being able to be moved by screwing or unscrewing on the threaded portion (11a) to pass from the proximal stop (17b) to the distal stop (17a), these stops being formed, respectively, by the two end walls of the housing (32) and wherein the edge (20') of the adjusting wheel (15') forms an index, and graduations (18) are provided on the body (3) of the housing (32).

6. An injection device according to any of the preceding claims, **characterised in that** the reservoir (5) is constituted by a standard injection cartridge or syringe.

7. An injection device according to one of the preceding claims, **characterised in that** the plunger rod (11) comprises an element that acts frictionally on the inner wall of the reservoir (5), such as especially an O-ring (22).

8. An injection device according to claim 1 comprising a reservoir (4), especially a cartridge or a syringe, the plunger (9) of which is moved by the plunger rod (11) which is of circular cross-section under the action of a control head (88, 90) and, at its distal end, an injection needle, the proximal end of the plunger rod (11) comprising means capable of successively cooperating, in a removable manner, with, on the one hand, a pressure plunger control head (88) and, on the other hand, a rotary plunger control head (90) and wherein the device comprises a nut (82) capable of being fixed to the proximal part of the reservoir (4), the diameter of the threaded hole of which nut is greater than that of the plunger rod (11) and is smaller than the internal diameter of the reservoir (4) **characterised in that** the pressure plunger control head (88) is constituted by a cylindrical element (89) of which the diameter is smaller than that of the threaded hole of the nut (82), so that it can be received in the reservoir (4), this cylindrical element (89) being capable of being fixed to the proximal end (86) of the plunger rod (11).

9. An injection device according to claim 8, **characterised in that** the rotary plunger control head (90) comprises a screw (98) able to be screwed into the threaded hole of the nut (82), so as to come to rest on the proximal part of the plunger rod (11) and be able to push it towards the distal end of the reservoir (4) when the screw (98) is screwed in and the screw (98) is fixed to the bottom of a cap (96).

10. An injection device according to any one of the preceding claims, **characterised in that** it comprises an injection needle (7) provided with a cannula (7a) hollowed out by a channel, this cannula (7a) being fixed to a base (36), comprising an axially centred circular plate (36a) with an adjacent cylindrical boss (36b) of the same axis (xx'), which is hollowed out by a cavity (36c) in communication with the channel of the needle, this cavity (36c) forming a convergent duct passing from its proximal part towards its distal part.

11. An injection device according to any one of claims 1 to 10, **characterised in that** it comprises an injection needle (7), provided with a cannula (7a), hollowed out by a channel, this cannula (7a) being integral with a cylindrical base (37) formed by a tubular element comprising a channel (7'') which is arranged in the extension of the channel of the cannula (7a), and the internal diameter (d2) of which is greater than that (d1) of the cannula and wherein the external diameter of the base (37) is greater than that of the cannula (7a).

FIG 1

FIG 3

FIG 1a

FIG 2

FIG 4

FIG 5

FIG 6

FIG 6a

20' position 3    20' position 2    20' position 1

v9  v8  v7  v6  v5  v4  v3  v2  v1

18

FIG 7

FIG 8

FIG 8a

FIG 8b

FIG 9

FIG 9a

FIG 10

FIG 10a

FIG 11

FIG 11a

FIG 12

FIG 13

FIG 14

FIG 15

FIG 16a

FIG 16c

FIG 16b

FIG 17

FIG 18

FIG 19

FIG 20

FIG 21

FIG 20a

**FIG 22**

**FIG 22a**

**FIG 23**

**FIG 23a**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2283915 A
- US 4189065 A
- FR 2953415
- WO 2007017052 A
- US 2011052303 A
- EP 0709040 A
- US 2005274746 A
- DE 102013212325
- US 8062254 B